(19) **Europäisches Patentamt**

**European Patent Office**

**Office européen des brevets**

(11) **EP 0 910 792 B1**

(12) **EUROPÄISCHE PATENTSCHRIFT**

(45) Veröffentlichungstag und Bekanntmachung des Hinweises auf die Patenterteilung:
**06.10.2004 Patentblatt 2004/41**

(51) Int Cl.$^7$: **G01N 21/64**, G01N 21/55, G01N 33/543

(21) Anmeldenummer: **97934432.2**

(22) Anmeldetag: **11.07.1997**

(86) Internationale Anmeldenummer:
**PCT/DE1997/001499**

(87) Internationale Veröffentlichungsnummer:
**WO 1998/002732 (22.01.1998 Gazette 1998/03)**

(54) **VORRICHTUNG UND VERFAHREN ZUR DURCHFÜHRUNG VON QUANTITATIVEN FLUORESZENZ MARKIERTEN AFFINITÄTSTESTS**

PROCESS AND DEVICE FOR CARRYING OUT QUANTITATIVE, FLUORESCENCE AFFINITY TESTS

PROCEDE ET DISPOSITIF POUR METTRE EN OEUVRE DES TESTS QUANTITATIFS D'AFFINITE A FLUORESCENCE

(84) Benannte Vertragsstaaten:
**AT BE CH DE DK ES FI FR GB GR IE IT LI NL SE**

(30) Priorität: **11.07.1996 DE 19628002**

(43) Veröffentlichungstag der Anmeldung:
**28.04.1999 Patentblatt 1999/17**

(73) Patentinhaber: **ICB INSTITUT FÜR CHEMO- UND BIOSENSORIK Münster e.V.**
**48149 Münster (DE)**

(72) Erfinder: **KATERKAMP, Andreas**
**D-34212 Melsungen (DE)**

(74) Vertreter: **Pfenning, Meinig & Partner**
**Mozartstrasse 17**
**80336 München (DE)**

(56) Entgegenhaltungen:
**WO-A-89/09408          WO-A-90/05295**
**WO-A-90/06503          WO-A-94/27137**
**US-A- 5 156 976          US-A- 5 192 510**

- **HLADY ET AL: "SPATIALLY RESOLVED DETECTION OF ANTIBODY-ANTIGEN REACTION ON SOLID/LIQUID INTERFACE USING TOTAL INTERNAL REFLECTION EXCITED ANTIGEN FLUORESCENCE AND CHARGE-COUPLED DEVICE DETECTION" BIOSENSORS & BIOELECTRONICS, Bd. 5, 1990, Seiten 291-301, XP002046044**

**Beschreibung**

Die Erfindung betrifft eine Vorrichtung zur Durchführung von insbesondere quantitativen Fluoreszenz markierten Affinitätstests mittels Evanescentfeldanregung. Dabei können verschiedenste bekannte biochemische Assays von allgemeinen Rezeptor-Ligand Systemen, wie z.B. Antikörper-Antigen, Lectin-Kohlenhydrat, DNA oder RNA-komplementäre Nukleinsäure, DNA oder RNA-Protein, Hormonrezeptor, Enzym-Enzymcofaktoren, Protein G oder Protein A-Immunglobin oder Avidin-Biotin zugrunde gelegt werden. Bevorzugt werden jedoch Antikörper-Antigen Systeme bewertet.

Fluoreszenzimmunotests oder auch Fluoreszenzimmunosensoren verwenden ein Antikörper-Antigen-Stystem und werden üblicherweise bereits seit längerer Zeit eingesetzt. Sie dienen dazu, hauptsächlich in einer flüssigen Probenmatrix eine unbekannte Menge einer bestimmten chemischen oder biochemischen Substanz zu quantifizieren. Dabei werden Antikörper selektiv an die zu bestimmende Substanz gebunden. Die zu bestimmende Substanz wird vom Fachmann auch als Antigen bezeichnet. Bei den Fluoreszenzimmunotests werden die analytspezifischen Antikörper mit einem Markierungsstoff markiert, der bei einer bestimmten stoffspezifischen Wellenlänge $\lambda_{ex}$ optisch angeregt wird und das Fluoreszenzlicht mit einer anderen Wellenlänge, die in der Regel größer ist, mit einem geeigneten Detektor unter Auswertung der Fluoreszenzlichtintensität genutzt. Die Ausnutzung der Evanescentfeldanregung bei der Durchführung solcher Fluoreszenzimmunotests respektive den Fluoreszenzimmunosensoren gehört bereits zum Stand der Technik. So sind verschiedene Lösungen bereits in WO 94/27137, von R.A. Badlay, R.A.L. Drake, I.A. Shanks, F.R.S., A.M. Smith und P.R. Stephenson in "Optical biosensors for immunoassays: fluorescence capillary-fill device", Phil. Trans. R. soc. Lund. B 316, 143 bis 160 (1987) und D. Christensen, S. Dyer, D. Fowers and J. Herron, "Analysis of Exitation and Collection Geometries for Planar Waveguide Immunosensors", Proc. SPIE-Int. Soc. Opt. Eng. Vol. 1986, Fiber Optic Sensors in Medical Diagnostics, 2 bis 8 (1993) beschrieben. Die bekannten Lösungen haben jedoch generell den Nachteil, daß sie einen relativ hohen Aufwand erforderlich machen, um das Licht, das zur Erzeugung der Fluoreszenz erforderlich wird, in einen Lichtleiter einzukoppeln bzw. das Fluoreszenzlicht auszukoppeln, die ein wesentlicher Bestandteil für die bisher üblicherweise verwendeten Vorrichtungen sind.

Daneben ist in US 3,939,350 eine Lösung beschrieben, bei der Fluoreszenzimmunoassays mittels Evanescentfeldanregung durchgeführt werden.

Dabei wird Licht einer Lichtquelle in einen Winkel durch ein Prisma auf eine Grenzfläche gerichtet, so daß Totalreflektion auftritt und die hervorgerufene Fluoreszenz in einer Probe mit einem Detektor gemessen werden kann. Das gesamte Probenvolumen ist hierbei in einem abgeschlossenen und abgedichteten Raum aufgenommen, so daß infolge des relativ großen Probenvolumens lediglich eine diffusionskontrollierte Endpunktdetektion erfolgen kann, die fehlerbehaftet ist.

In WO 90/05295 ist ein optisches Biosensorsystem beschrieben, bei dem eine aufwendige Optik Anregungslicht auf sensitive Bereiche eines ebenfalls aufwendigen Kanalsystems, durch das Probenvolumen mittels gesteuerten Ventilen und Pumpen geführt wird, gerichtet werden kann und das durch Fenster aus den sensitiven Bereichen austretende Fluoreszenzlicht wieder auf einen Detektor zur Intensitätsmessung gerichtet werden kann. Neben dem bereits erwähnten nachteiligen komplizierten und aufwendigen Aufbau erfordert dieses System vor bzw. nach Durchführung eines Tests eine Reinigung sowohl der Pumpen, wie auch des gesamten Kanalsystems, um nachfolgende Meßfehler ausschließen zu können.

In WO 90/06503 ist ein Sensor beschrieben, bei dem Anregungslicht in einem geeigneten Winkel durch ein optisch transparentes Substrat auf eine Grenzfläche zu einer optisch. transparenten Pufferschicht gerichtet wird, über der eine zusätzliche Wellenleiterschicht aufgebracht ist, an der wiederum die zu bestimmenden Analyten gebunden werden.

Der Brechungsindex der Pufferschicht ist dabei kleiner als der des Substrates und des Wellenleiters. An der Grenzschicht Substrat/Puffer erfolgt Totalreflektion durch geeignete Wahl des Winkels des Anregungslichtes und über das dabei entstehende evanescente Feld wird das Anregungslicht in den über der Pufferschicht befindlichen Wellenleiter eingekoppelt. Das eingekoppelte Licht in den Wellenleiter wird über Totalreflektion im Wellenleiter geführt und das dabei sich ausbildende evanescente Feld wird entsprechend zur Fluoreszanregung genutzt.

Die Probe kann in einem oder mehreren Hohlräumen aufgenommen sein, wobei die entsprechende Dimensionierung eines solchen Hohlraumes nur dahingehend eingeschränkt wird, daß seine Größe den Probentransport in den Hohlräumen mittels Kapillarkraft ermöglicht. Nach der Probenaufnahme in den Hohlräumen erfolgt kein weiterer Fluß oder Bewegung der Probe.

Aus WO 89/09408 A1 ist eine ähnliche Lösung zu entnehmen, die wiederum die Lichtquelle für das Anregungslicht und den Detektor für das Fluoreszenzlicht auf der gleichen Seite verwendet. Die zu detektierende Probe wird in einem Hohlraum zwischen einem Wellenleiter und einer Deckplatte aufgenommen. auch hier erfolgt nach dder Probennahme kein weiterer Fluß oder Bewegung der Probe.

Es ist daher Aufgabe der Erfindung, eine Möglichkeit zu schaffen, mit einer sehr einfach aufgebauten Vorrichtung quantitative Fluoreszenz markierte Affinitätstests mit verschiedenen bekannten biochemischen Assays durchführen zu können.

Erfindungsgemäß wird diese Aufgabe durch die im kennzeichnenden Teil des Anspruchs 1 enthaltenen Merkmale für die Vorrichtung und den Merkmalen des Anspruchs 25 für das Verfahren gelöst. Vorteilhafte Ausgestaltungsformen und Weiterbildungen der Erfindung ergeben sich mit der Verwendung der in den untergeordneten Ansprüchen enthaltenen Merkmale.

Mit der erfindungsgemäß ausgebildeten Vorrichtung können Fluoreszenzimmunotests in verschiedenen Prozeßabläufen (Assays) und weitere quantitative Fluoreszenz markierte Affinitätstests durchgeführt werden. So besteht einmal die Möglichkeit der Durchführung von kompetitiven Assays und es können aber auch Sandwichassays und andere bekannte Assayformen genutzt werden.

Mit der erfindungsgemäßen Vorrichtung wird ähnlich gearbeitet, wie dies bereits im Stand der Technik bekannt ist. Dabei wird als Markierungsstoff ein Fluorophor verwendet und mit diesen analytspezifischen Antikörper markiert. Der gebundene Fluorophor mit einer Evanescentfeldanregung angeregt und die Fluoreszintensität, die dadurch hervorgerufen worden ist, ermöglicht die Quantifizierung der markierten Antikörper und so wird auch der Analyt quantifizierbar.

Bei der Vorrichtung nach der Erfindung wird Licht einer Lichtquelle unter einem Winkel $\alpha$ auf die Grenzfläche zweier Medien mit unterschiedlichem Brechungsindex gerichtet. Dabei wird eine Lichtquelle ausgewählt, die nahezu monochromatisches Licht mit einer Wellenlänge aussendet, die geeignet ist, den Markierungsstoff, in diesem Fall das Fluorophor, anzuregen. Als Lichtquelle eignen sich dabei insbesondere Laserdioden, da diese ein geeignetes Strahlprofil und eine ausreichende Lichtleistung, bei kleiner Baugröße und geringem Energieverbrauch aufweisen.

Es können aber auch andere monochromatisches Licht aussendende Lichtquellen Verwendung finden.

Dabei ist im folgenden zu beachten, daß sämtliche optisch transparenten Elemente jeweils bei den verwendeten Wellenlängen transparent sind.

Der Winkel $\alpha$ mit dem das ausgesendete Licht auf die Grenzfläche gesendet wird, bestimmt neben dem Brechungsindex, des im Strahlengang vor der Grenzfläche angeordneten Materials und dem sich daran anschließenden Material gemeinsam mit der Wellenlänge des Lichtes, die Eindringtiefe d für das evanescente Feld. Dabei muß der Brechungsindex $n_1$ des Materials, das im Strahlengang vor der Grenzfläche angeordnet ist Totalreflexion an der Grenzfläche ermöglichen und sollte daher größer als der Brechungsindex $n_2$ des anderen, danach angeordneten Materials, bei den Wellenlängen der verwendeten Lichtquellen sein. Der Winkel $\alpha$ wird bevorzugt so gewählt, daß gilt: $\sin(\alpha) > n_2/n_1$. Wird diese Voraussetzung erfüllt, wird alles Licht an der Grenzfläche reflektiert und somit Totalreflexion erreicht. Bei Erfüllung dieser Bedingung, dringt jedoch ein relativ kleiner Teil des Lichts durch die Grenzfläche in das Material, das im Strahlengang nach der Grenzfläche angeordnet ist, ein und das evanescente Feld entsteht. Die Eindringtiefe d wird dadurch definiert, daß sie der Entfernung von der Grenzfläche entspricht, bei der die Intensität des evanescenten Feldes auf den Wert 1 durch e abgefallen ist, wobei e gleich der Basis des natürlichen Logarithmus ist. Die Eindringtiefe d läßt sich mit der folgenden Gleichung berechnen:

$$d = \frac{\lambda}{2\pi} * \frac{1}{\sqrt{n_1^2 * \sin^2(\alpha) - n_2^2}}$$

Mit einer Wellenlänge von $\lambda$ = 700 nm, einem Brechungsindex von $n_1$ = 1,51 und einem Brechungsindex von $n_2$ = 1,34 wird bei einem Einfallswinkel des Lichtes von $\alpha$ = 65° eine Eindringtiefe d von ca. 400 nm und bei einem Einfallswinkel $\alpha$ = 80° eine Eindringtiefe d von ca. 173 nm erreicht. Daraus folgt, daß durch das evanescente Feld nur solche Markierungsstoffe optisch angeregt werden können, die sich in unmittelbarer Nähe der Grenzfläche befinden. Für die Durchführung der Fluoreszenzimmunotests ergibt sich daraus, daß ausschließlich die Markierungsstoffe der Antikörper oder Antigene angeregt werden, die auf der Oberfläche der Grenzfläche gebunden sind. Die Fluoreszenzintensität des von diesen Fluorophoren emittierten Lichtes ist damit direkt proportional zur Konzentration der an der Oberfläche gebundenen markierten Antikörper und je nach dem benutzten biochemischen Assay proportional oder umgekehrt proportional zur Antigenkonzentration.

Die erfindungsgemäß ausgebildete Vorrichtung verwendet nunmehr mindestens eine Lichtquelle, die nahezu monochromatisches Licht aussendet und dieses in einem, die Eindringtiefe d für das evanescente Feld vorgebenden Winkel $\alpha$ auf eine für dieses Licht transparente Bodenplatte richtet. Der Brechungsindex $n_1$ der Bodenplatte sollte größer als 1,33 sein. Auf der anderen Seite der Bodenplatte wird zwischen einer Deckplatte ein küvettenförmig ausgebildeter Aufnahmebereich ausgebildet. Zwischen Bodenplatte und dem küvettenförmig ausgebildeten Aufnahmebereich ist besagte Grenzfläche ausgebildet und das evanescente Feld kann sich mit der vorgegebenen Eindringtiefe d innerhalb des küvettenförmigen Aufnahmebereiches auf an die Oberfläche gebundenen markierten chemischen oder biochemischen Partner eines Rezeptor-Lignad Systems auswirken und die als Markierungsstoff verwendeten Fluorophore anregen.

Die so hervorgerufene Fluoreszenz wird mit der entsprechenden Intensität mit einem Detektor gemessen. Der Detektor ist dabei an der gleichen Seite der Bodenplatte, wie die Lichtquelle angeordnet.

Als Detektor kann dabei ein einzelner lichtempfindlicher Detektor, eine lineare oder eine flächenhafte Anordnung von mehreren lichtempfindlichen Detektoren verwendet werden.

Obwohl, bei der Verwendung von Laserdioden das gesendete Licht nahezu monochromatisch ist, wird ein schwacher spektralbreiter Fluoreszenzuntergrund im Laserlicht beobachtet. Aus diesem Grunde wird vorzugsweise zwischen Lichtquelle und Bodenplatte ein schmalbandiger Anregungsfilter angeordnet, der bevorzugt eine Spektralbandbreite < 10 nm hat und nur Licht in diesem engen begrenzten Wellenlängenbereich angepaßt an die Lichtquelle durchläßt.

Vorteilhaft kann auch ein zweiter relativ breitbandiger Filter (Emissionsfilter) vor dem Detektor angeordnet werden. Dieser Filter verhindert, daß im Material der Bodenplatte und bei der Reflexion an der Grenzfläche gestreutes Licht der Lichtquelle auf den Detektor gelangt und das Meßergebnis verfälscht. Für die beiden Filter bieten sich günstigerweise Interferenzfilter oder Kantenfilter oder eine Kombination aus beiden an.

Besonders vorteilhaft ist es, polarisiertes Licht auf die zu bestimmende Probe zu richten. Hierfür kann in den Strahlengang des Lichtes im Anschluß an die Lichtquelle ein Polarisator angeordnet werden.

Bei polarisierter Anregung und Detektion wird in günstigerweise ausgenutzt, daß die Fluorophore der markierten Antikörper, die an der Oberfläche fest gebunden sind, in ihrer Beweglichkeit gehemmt sind. Demzufolge ist deren Fluoreszenzlicht genau wie das Anregungslicht in der gleichen Polarisationsebene ausgerichtet und kann detektiert werden.

Im Gegensatz dazu wird das Fluoreszenzlicht der Fluorophore der chemischen oder biochemischen Substanz (Antikörper), die nicht an der Oberfläche gebunden sind und sich demzufolge frei bewegen können, in anderen Polarisationsebene ausgerichtet sein. Mit der Verwendung von polarisiertem Licht zur Anregung kann das Fluoreszenzlicht der an den Antikörper oder Antigene gebundenen Fluorophore unterdrückt werden, die sich zwar in Reichweite des evanescenten Feldes befinden, jedoch nicht an die Oberfläche gebunden sind. Der Vorteil der polarisierten Anregung und Detektion wird verständlich, wenn die Abmessungen eines Antikörpers von ca. 10 nm ins Verhältnis zur Eindringtiefe des evanescenten Feldes bis zu etwa 500 nm betrachtet wird. Die Polarisation bei der Anregung und Detektion, wobei dafür auch vor dem Detektor ein Polarisator angeordnet wird, kann das Verhältnis von Nutzsignal zu Hintergrundsignal verbessern.

Die erfindungsgemäß ausgebildete Vorrichtung weist gegenüber den bisher verwendeten einige wesentliche Vorteile auf. Sie verfügt über einen sehr einfachen Aufbau, der geringe Ansprüche an die zu verwendenden optischen Bauelemente stellt und insbesondere bei der Einkopplung des Lichtes zur Anregung der Fluorophore und/oder bei der Auskopplung des Fluoreszenzlichtes keine gesonderten Anforderungen stellt. Außerdem können die verschiedensten biochemischen Assays ohne weiteres durchgeführt und die wesentlichen Bestandteile kostengünstig hergestellt werden, so daß auch ein einmaliger Gebrauch zumindest von Teilen ohne weiteres möglich ist. Außerdem wird die notwendige Separation der chemischen Komponenten aus dem Probenvolumen, von den an der Oberfläche gebundenen Komponenten, gewährleistet.

Dies wird auch dadurch erreicht, daß Bodenplatte und Deckplatte sowie die dazwischen angeordneten Abstandshalter aus einfachen und kostengünstig beschaffbaren Materialien, die mit Standard Technologien bearbeitet werden können, bestehen. So können für Boden- und Deckplatte Kunststoffe und für den Abstandshalter in günstiger Form ein biokompatibler Klebefilm eingesetzt werden, der beidseitig haftend ausgebildet ist.

Der Abstandshalter weist eine Dicke von 0,001 bis 10 mm, bevorzugt zwischen 0,001 und 0,5 mm und besonders bevorzugt 50 µm auf und bildet durch eine Aussparung den Aufnahmebereich für die Probe aus.

Das erfindungsgemäße Verfahren beruht im wesentlichen darauf, daß ein definiertes Probenvolumen durch den küvettenförmigen Aufnahmebereich geführt und dort einer Evanescentfeldanregung unterzogen wird, wie dies bereits beschrieben wurde. Das Probenvolumen kann dabei durch Saug-, Druckwirkung Kappilarkräften durch den küvettenförmigen Aufnahmebereich geführt werden.

Bei der Anbindung einer chemischen oder biochemischen Komponente an seiner auf einer Oberfläche immobilisierten komplementären chemischen oder biochemischen Komponente müssen in einem strömenden System zwei Stofftransport-Effekte, nämlich Konvektion und Diffusion berücksichtigt werden. Dabei beeinflußt die Dicke einer diffusiven Grenzschicht (strömungsgeschwindigkeits- und viskositätsabhängig) die Anbindung wesentlich in Bezug auf die Quantität und Geschwindigkeit. Überwiegt der diffusive Stofftransport bei relativ dicker diffusiver Grenzschicht kann auch eine Rückbindung bereits gebundener chemischer oder biochemischer Komponenten erfolgen, die das Meßergebnis verfälscht. Dem kann mit der Erfindung entgegengewirkt werden, in dem der freie Querschnitt des küvettenförmigen Aufnahmebereiches insbesondere in der Höhe relativ klein gehalten wird. Unter Beibehaltung der Strömungsgeschwindigkeit, führt dies in Zusammenwirkung dazu, daß die diffusive Grenzschicht im eigentlichen Detektionsbereich vernachlässigbar klein ist. Dadurch kann die Messung wesentlich schneller mit ausreichender ggf. sogar höherer Genauigkeit durchgeführt werden. In vielen Fällen kann sogar auf eine Endpunktdetektion verzichtet werden, daß heißt es muß nicht unbedingt das gesamte Probenvolumen berücksichtigt werden.

In einer vorteilhaften Ausführung ist in einer Deckplatte zumindest eine Anschlußmöglichkeit, die zumindest teilweise eine Öffnung ist, vorgesehen, in die ein Probencontainer einsetzbar oder angeordnet ist, dabei ist die Öffnung in der Deckplatte so angeordnet, daß eine Verbindung zwischen Probencontainer und Aufnahmebereich herstellbar ist. Zusätzlich ist eine zweite Öffnung als weitere solch eine Anschlußmöglichkeit vorhanden, die ebenfalls mit dem küvettenförmigen Aufnahmebereich verbunden ist.

Die zweite Öffnung kann ebenfalls in der Deckplatte vorgesehen sein. An diese zweite Öffnung kann eine externe Pumpe angeschlossen oder eine eigene Pumpe eingesetzt werden. Die eigene Pumpe besteht dabei bevorzugt aus einem zylinderförmigen Hohlkörper, der passend in die zweite Öffnung einsetzbar ist. Am Boden des zylinderförmigen Hohlkörpers ist ein saugfähiges Material, beispielsweise ein Vlies oder Papier angeordnet. Der zylindrische Hohlkörper kann mit einem Stöpsel, einer Kappe oder einer Folie abgeschlossen sein. Durch Entfernen des Stöpsels oder Kappe sowie Einbringen einer Öffnung in der Folie wird der Pumpvorgang aktiviert.

Die Erfindung kann man weiter ausbilden, in dem in den bereits bezeichneten Probencontainer eine Hülse einsetzbar ist, die nach unten mit einer Membran abgeschlossen sein kann. Dabei können auf der Membranvorzugsweise Antigene immobilisiert sein und an der Innenwandung der Hülse befinden sich für die Durchführung des Testes erforderliche markierte analytspezifische Antikörper.

Nachfolgend soll die Erfindung beispielhaft näher beschrieben werden.

Dabei zeigt:

Fig. 1     das Meßprinzip eines kompetitiven Assays;

Fig. 2     das Meßprinzip einer Fluoreszenzmessung mit Evanescentfeldanregung von oberfläche gebundenen markierten Antikörpern;

Fig. 3     das Meßprinzip der Durchführung eines Sandwichassays;

Fig. 4     das Prinzip zur Durchführung eines verbesserten kompetitiven Assays;

Fig. 5     einen Teil der erfindungsgemäßen Vorrichtung zur Durchflußanordnung;

Fig. 6     eine schematische Darstellung einer Ausführungsform einer erfindungsgemäß ausgebildeten Vorrichtung mit einer Lichtquelle;

Fig. 7     eine schematische Darstellung einer zweiten Ausführungsform mit zwei verwendeten Lichtquellen;

Fig. 8     eine schematische Darstellung einer weiteren Ausführungsform mit einer Lichtquelle;

Fig. 9     ein weitergebildetes Beispiel nach Fig. 6;

Fig. 10    die Anordnung eines Probencontainers und einer Pumpe an der erfindungsgemäßen Vorrichtung;

Fig. 11    eine Weiterbildung der Vorrichtung nach Figur 10 mit einer zusätzlich einsetzbaren Hülse und

Fig. 12    eine weitere Ausführungsform einer Bodenplatte mit Abstandshalter.

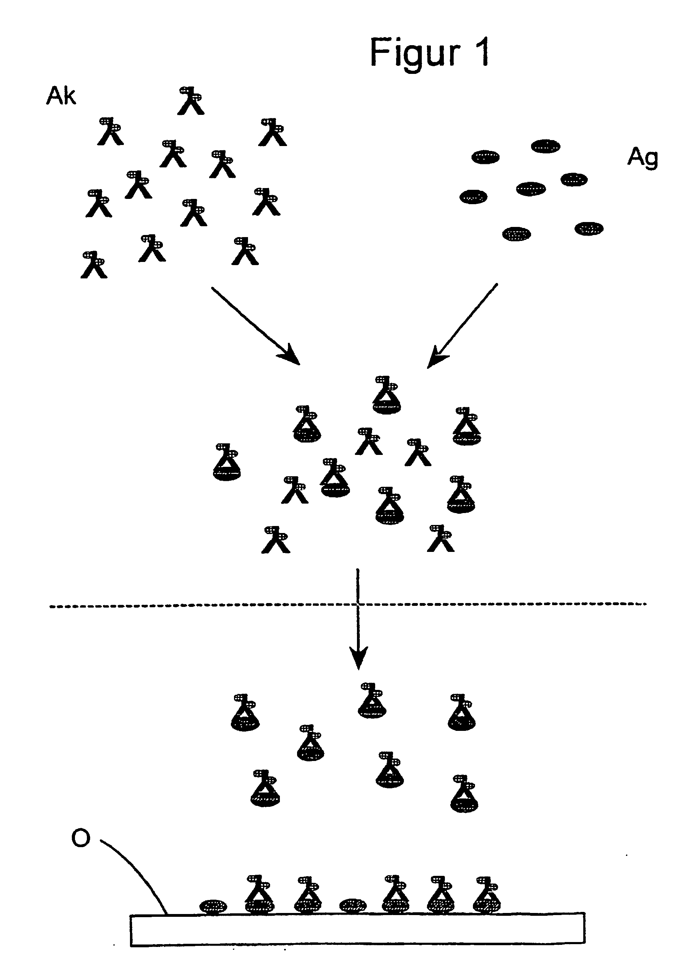

Der Figur 1 kann schematisch das Meßprinzip für die Durchführung eines kompetitiven Assays entnommen wedren. Eine bekannte Menge Fluorophor markierter analytspezifischer Antikörper Ak wird mit einer unbekannten zu quantifizierenden Menge Antigenen Ag gemischt, dabei muß die Menge der Antikörper Ak größer sein, als die Menge der Antigene Ag. Die markierten analytspezifischen Antikörper Ak binden an die Antigene Ag und, da die Menge der markierten analytspezifischen Antikörper Ak größer als die der Antigene Ag ist, bleiben ungebundene markierte analytspezifische Antikörper Ak über. Das so entstandene Gemisch aus nicht gebundenen bzw. freien markierten analytspezifischen Antikörpern Ak wird mit einer Oberfläche (O) in Kontakt gebracht, auf der das Antigen Ag immobilisiert wurde. Zur besseren Verdeutlichung ist diese Phase des Assays durch die gestrichelte Linie in der Darstellung getrennt. An die immobilisierten Antigene Ag binden die noch freien markierten analytspezifischen Antikörper Ak an, während die schon gebundenen in der Lösung oberhalb der Oberfläche verbleiben. Die Menge der analytspezifischen Antikörper Ak, die an das auf der Oberfläche immobilisierte Antigen Ag gebunden sind, ist umgekehrt proportional zur Antigenkonzentration, die nachgewiesen werden soll.

Die Menge, der an der Oberfläche gebundenen markierten analytspezifischen Antikörper Ak kann durch Evanescentfeldanregung und Messung der Fluoreszenzintensität quantifiziert werden.

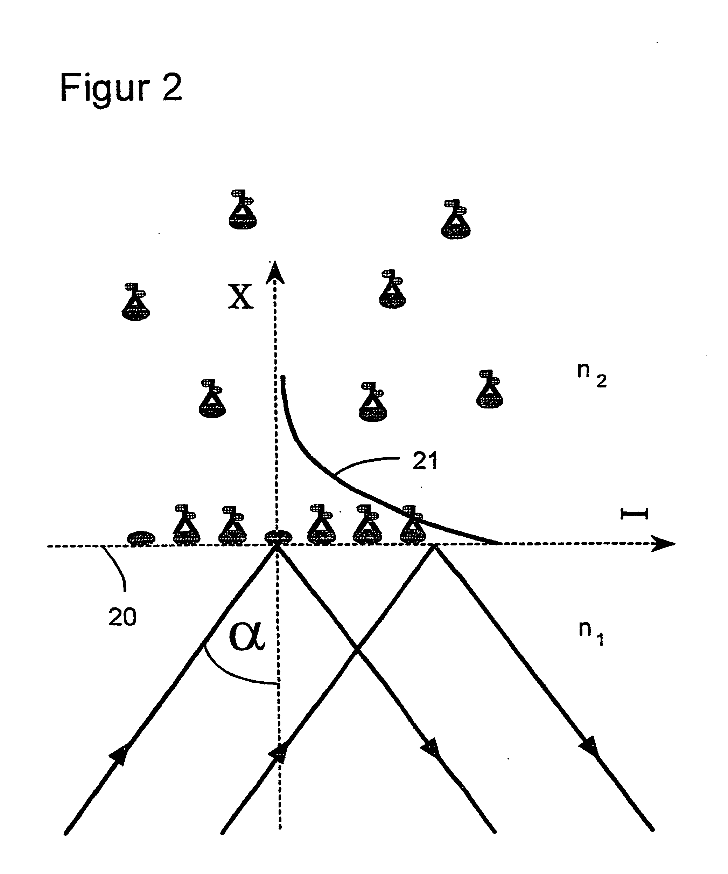

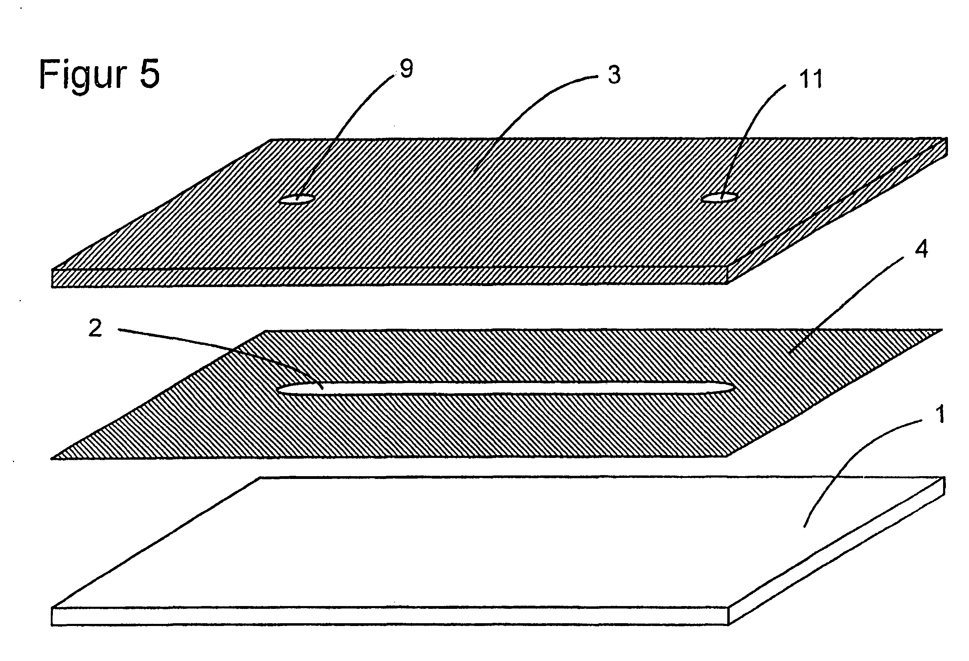

Dieses Meßprinzip kann der Figur 2 entnommen werden. Dabei wird durch ein Medium mit dem Brechungsindex $n_1$, das später bei der Beschreibung der Figuren 5 bis 9 als Bodenplatte 1 bezeichnet wird, Licht mit einer Wellenlänge, mit der das Fluorophor als Markierungsstoff angeregt werden kann, in einem Winkel $\alpha$ parallel gegen die als Grenzfläche 20 wirkende Oberfläche, auf der die immobilisierten Antigene gebunden sind, gerichtet. Das Medium mit dem Brechungsindex $n_1$ ist hier unter der Grenzfläche 20 angeordnet.

An dieser als Grenzfläche 20 dienenden Oberfläche erfolgt Totalreflexion und es bildet sich ein evanescentes Feld oberhalb dieser Oberfläche aus, das die Anregung der Fluorophore bewirkt.

Der Figur 2 ist dabei auch die Intensität I mit dem Kurvenverlauf 21 des evanescenten Feldes in Abhängigkeit des Abstandes X von der als Grenzfläche 20 wirkenden Oberfläche dargestellt, wobei eindeutig zu erkennen ist, daß sich die Intensität 21 mit wachsendem Abstand exponentiell verringert.

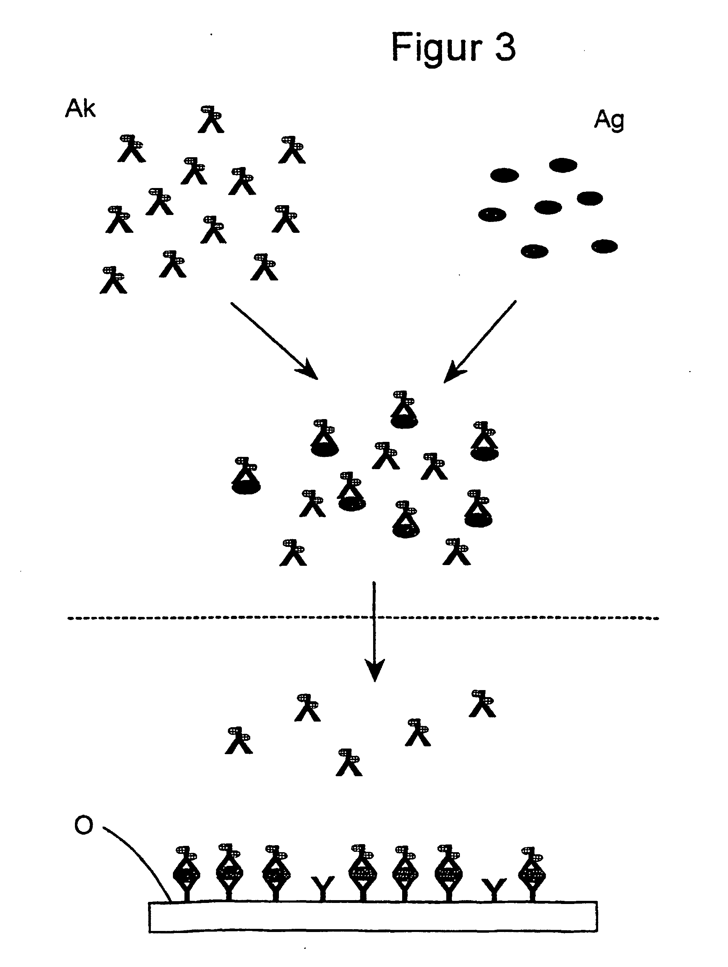

Der Figur 3 ist schematisch der Ablauf eines anderen Assayformates, eines sogenannten Sandwichassays zu entnehmen, wobei die Phasen wieder durch die eingezeichnete gestrichelte Linie getrennt sind. Dabei ist Voraussetzung, daß ein Paar von analytspezifischen Antikörpern Ak existiert, die beide gleichzeitig an das Antigen Ag binden können, ohne daß diese analytspezifischen Antikörper Ak sich gegenseitig behindern. Dabei wird einer der beiden analytspezifischen Antikörper mit einem Fluorophor markiert. Der andere der analytspezifische Antikörper Ak ist auf einer Oberfläche immobilisiert. Nach der Reaktion und Oberflächenanbindung des zweiten Antikörpers werden die Fluorophore bei Bestrahlung mit Licht einer bestimmten Wellenlänge dann angeregt und es wird ein Fluoreszenzsignal erhalten, das bei dieser Form des Immunotests direkt proportional zur Antigenkonzentration ist. Das hat den Vorteil, daß bereits eine kleinere Antigenkonzentration ein erfaßbares Signal erzeugt und auch kleine Änderungen in der Antigenkonzentration empfindlich erkannt werden können.

Demgegenüber hat das bereits beschriebene kompetitive Assay (vergl. Fig. 1) den Nachteil, daß kleine Antigenkonzentrationen bereits ein großes Signal erzeugen und entsprechend kleine Änderungen nur schwer gemessen werden können.

Es ist allgemein bekannt, daß beim sandwichassay die Nachweisgrenze kleiner und die Empfindlichkeit größer als beim kompetitiven Assay ist. Der Sandwichassay hat aber demgegenüber den Nachteil, daß ein zweiter analytspezifischer Antikörper Ak existieren muß und dies nur dann möglich ist, wenn die Molmasse des Antigens Ag wesentlich größer als 200 Dalton ist. Es können demzufolge niedermolekulare Antigene, wie beispielsweise Umweltschadstoffe so nicht bzw. nur schwer nachgewiesen werden.

Daraus folgt, daß kompetitive Assays universeller einsetzbar sind, jedoch eine geringere Empfindlichkeit haben, wohingegen der Sandwichassay in diesen Punkten zwar besser ist, jedoch nicht in allen Fällen eingesetzt werden kann.

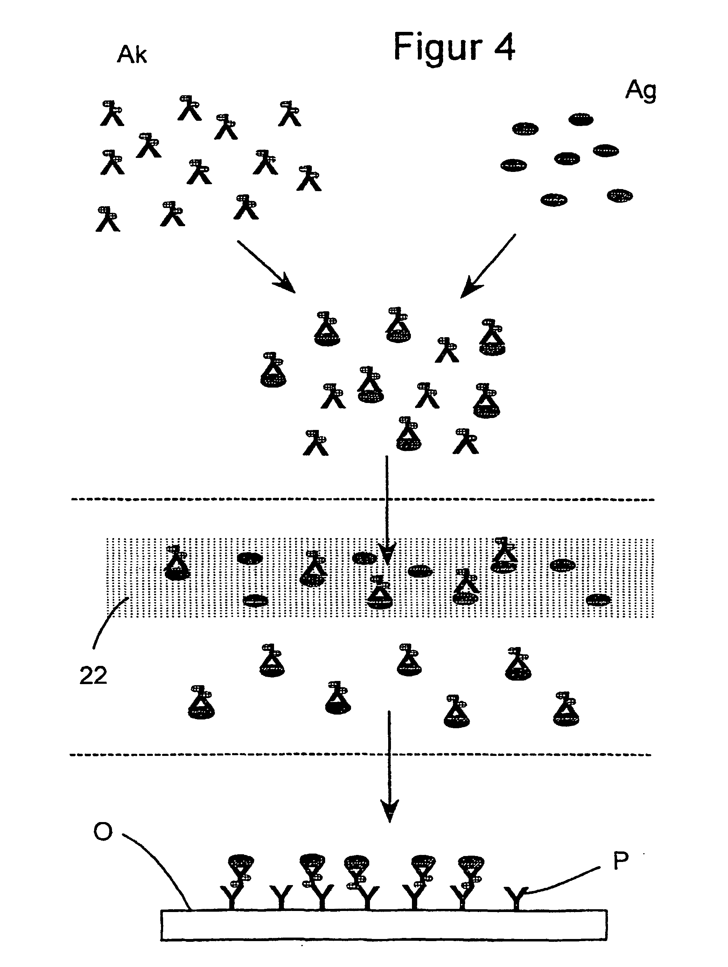

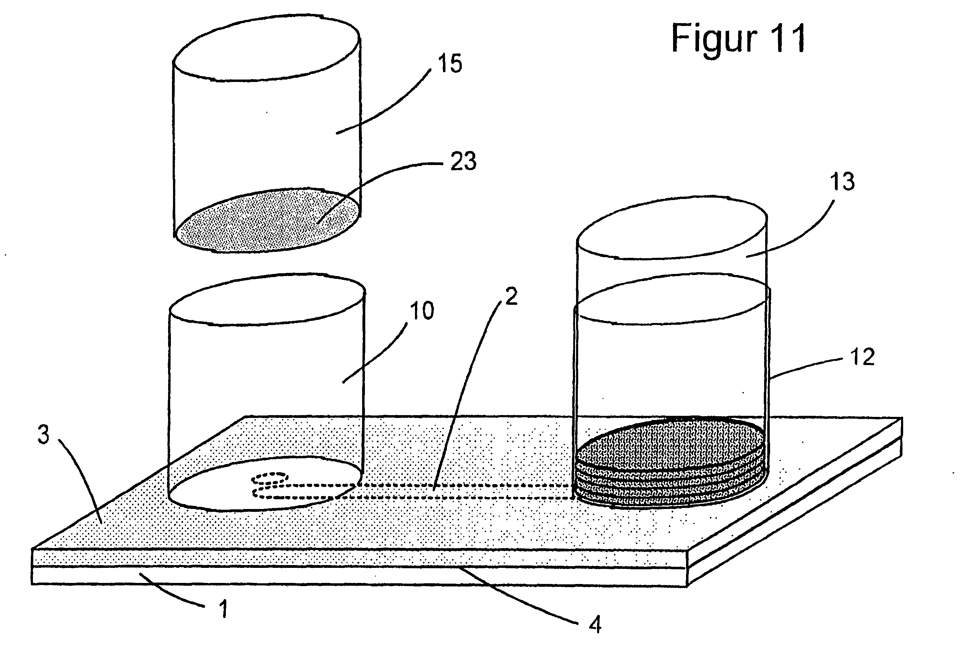

In Figur 4 ist das Prinzip eines neuartigen Assays beschrieben, wobei hierzu noch nähere Ausführungen, insbesondere für die zu verwendende Vorrichtung bei der Beschreibung der Figur 11 erfolgen sollen.

Die Basis für diesen neuartigen Assay bildet wieder der kompetitive Assay. Das Gemisch aus freien und gebundenen markierten analytspezifischen Antikörpern Ak wird durch eine Membran 22, auf deren Oberfläche das entsprechende Antigen Ag immobilisiert ist, geführt. Als Membranmaterial kann beispielsweise eine Nitrozellulosemembran verwendet werden. An die dort immobilisierten Antigene Ag binden ausschließlich die freien markierten analytspezifischen Antikörper Ak an, während die bereits gebundenen die Membran 22 passieren. Die Flüssigkeit im Anschluß an die Membran 22 wird mit einer Oberfläche O in Kontakt gebracht, auf der ein Protein P immobilisiert ist. Dieses Protein ist in der Lage unabhängig von der Spezifizität des verwendeten markierten analytspezifischen Antikörpers Ak, diesen zu erkennen und zu binden. Als Protein P kann beispielsweise Protein A oder ein anti-Antikörper eingesetzt werden. Das bei diesem Assay erhaltene Fluoreszenzintensitätssignal verhält sich entsprechend dem Sandwichassay und die Empfindlichkeit ist demzufolge größer und die mögliche Nachweisgrenze entsprechend klein.

In der Figur 5 ist der prinzipielle Aufbau eines Teiles der erfindungsgemäßen Vorrichtung dargestellt. Die dort abgebildeten drei Teile, die Bodenplatte 1, der Abstandshalter 4 und die Deckplatte 3, können vor der Durchführung des Fluoreszenzimmunotestes miteinander verbunden werden oder bilden bereits eine fertig komplettierte Einheit und gleichen in ihrem Aufbau einer Durchflußzelle und einer Meßküvette.

Dabei besteht die Bodenplatte 1 aus einem hochbrechenden transparenten Material, wie beispielsweise Glas oder einem Kunststoff, wie einem Polymer (PMMA oder PC) mit einem Brechungsindex $n_1 > 1,33$. Die Dicke der Bodenplatte kann in einem Bereich von 0,01 bis 10 mm, bevorzugt zwischen 0,5 und 1 mm liegen.

Der Abstandshalter 4 ist bevorzugt eine dünne Folie, die beidseitig mit einem Klebefilm versehen ist, oder ein dünner Klebefilm und so einmal auf die Bodenplatte 1 und zum anderen auf die Deckplatte 3 klebbar. Die gesamte Dicke des Abstandshalters inklusive des verwendeten Klebemittels sollte in einem Bereich bevorzugt zwischen 0,001 und 0,5 mm und ganz besonders bevorzugt bei einer Dicke von 50 µm liegen. In den Abstandshalter 4 ist eine Durchbrechung herausgearbeitet, die einen küvettenförmigen Aufnahmebereich 2 ausbildet.

In der Figur 5 ist außerdem die Deckplatte 3 in der durchgehende Öffnungen 9 und 11, bei diesem Beispiel als Bohrungen ausgebildet, erkennbar. Auf deren Funktion wird später noch zurückzukommen sein. Die Öffnungen 9 und 11 sind dabei so angeordnet, daß sie zumindest teilweise den Bereich des Aufnahmebereiches 2 des Abstandshalters 4 übergreifen. Der Abstandshalter 4 kann vorzugsweise auch aus einem biokompatiblen Klebefilm, der bevorzugt beidseitig mit einer abziehbaren Schutzschicht versehen ist und bereits kommerziell erhaltbar ist, bestehen.

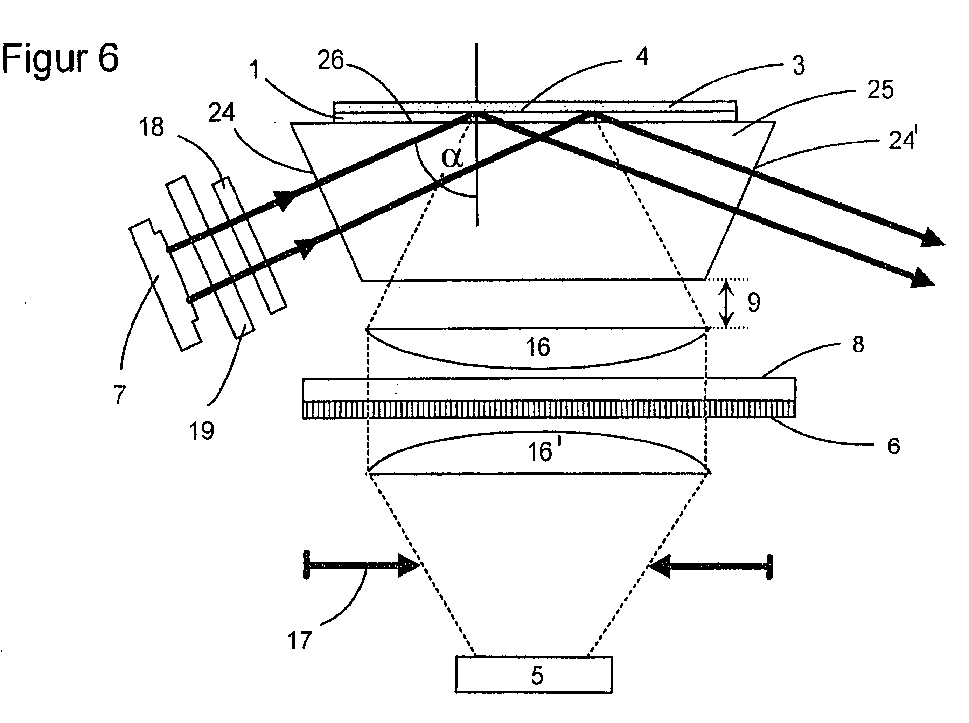

In der Figur 6 ist der prinzipielle Aufbau einer erfindungsgemäßen Vorrichtung erkennbar. Dabei wird Licht einer Laserdiode 7 durch einen Polarisator 18, durch einen Anregungsfilter 19, der optisch schmalbandig ausgebildet ist, auf den im Abstandshalter 4 ausgebildeten Aufnahmebereich 2 durch die Bodenplatte 1 gerichtet. Durch die Totalreflexion an der Grenzfläche 20 zwischen Bodenplatte 1 und Aufnahmebereich 2 erfolgt im Aufnahmebereich 2 eine Evanescentfeldanregung, die Fluoreszenz der als Markierungsstoff verwendeten Fluorophore hervorruft. Das Fluoreszenzlicht gelangt von dort über eine Linse 16 durch ein breitbandiges Filter 8, mit dem Streulicht der Laserdiode 7 vom Detektor 5 ferngehalten wird. Das Fluoreszenzlicht gelangt durch den nachgeordneten Polarisator 6 mit Hilfe der Linse 16' auf den Detektor 5, dem eine Blende 17 vorgeschaltet ist. Mit dem Detektor 5 wird die Fluoreszenzintensität erfaßt und demzufolge der Fluoreszenzimmunotest durchgeführt und eine entsprechende quantitative Bestimmung ermöglicht.

Bei dem in Figur 6 gezeigten Beispiel, wird vorteilhaft das Licht der Laserdiode 7, wie bereits beschrieben, über den Polarisator 18 und einen Anregungsfilter 19 durch einen optisch transparenten Körper 25 und die Bodenplatte 1 auf die Grenzfläche 20 gerichtet. Selbstverständlich kann auch das Licht unter Wegfall des Polarisators 18 und Anregungsfilters 19 direkt auf eine Stirnfläche 24 des transparenten Körpers 25 gerichtet sein. Der transparente Körper 25 besteht aus einem Material mit einem höheren Brechungsindex als ihn das Material, das oberhalb der Grenzfläche 20 angeordnet ist, hat. Bevorzugt weist der transparente Körper 25 den gleichen Brechungsindex, wie die Bodenplatte 1 auf.

Günstigerweise wird als transparenter Körper 25 ein Körper, der z.B. wie ein abgeflachtes Glasprisma oder Kunststoffprisma ausgebildet ist verwendet, wobei verschiedene Kunststoffe, mit dem genannten Brechungsindex und anderen günstigen optischen Eigenschaften zu verwenden sind.

Der transparente Körper 25 kann einmal mit einem Kleber mit der Bodenplatte 1 verbunden sein, wobei bevorzugt ein Kleber mit gleichen optischen Eigenschaften verwendet werden soll. Eine weitere Möglichkeit besteht darin, um einen optimalen optischen Kontakt zwischen dem hochbrechenden optisch transparenten Körper 25 und der Bodenplatte 1 zu erhalten, einen sehr dünnen Film 26 (Matching Fluid) zwischen diesen beiden einzubringen, wobei im günstigsten Fall der Brechungsindex des Fluids mit den Brechungsindizes der Bodenplatte 1 und dem transparenten Körper 25 übereinstimmt. Kleber oder Film bilden eine optische Schicht 26 zur Vermittlung des optisches Kontaktes zwischen dem Körper 25 und der Bodenplatte 1.

Die Verwendung des transparenten Körpers 25 führt dazu, daß der größte Teil des Lichtes aus der Laserdiode 7 zur Totalreflexion an der Grenzfläche 20 zwischen der Bodenplatte 1 und dem Aufnahmebereich 2 gelangen kann.

Dabei ist es besonders günstig, die Stirnfläche 24 oder 24' so zu gestalten und auszurichten, daß das Licht der Laserdiode 7 orthogonal auf diese Stirnfläche 24 oder 24' auftrifft und so eine maximale Lichtausbeute zur Erreichung der Totalreflexion an der Grenzfläche 20, einkoppelbar ist.

Im einfachsten Fall kann die Bodenplatte 1 und der transparente Körper 25 als ein gemeinsames Bauteil ausgebildet sein, so daß auf die bereits beschriebene Verbindung bzw. die Verwendung des sehr dünnen Films 26 zwischen transparentem Körper 25 und Bodenplatte 1 verzichtet werden kann.

Bei diesem Beispiel wird vorteilhaft auch ein Kollimator (Linse) 16, 16' verwendet, mit dem das Fluoreszenzlicht konzentriert auf den Detektor 5 gerichtet werden kann. Bei dem gezeigten Beispiel besteht der Kolimator aus zwei getrennten Linsen 16 und 16', die sich gegenüberliegend angeordnet sind und zwischen denen sich der Filter 8 und der Polarisator 6 befinden kann. Selbstverständlich kann aber auch eine einteilige Linse als Kollimator 16 verwendet werden.

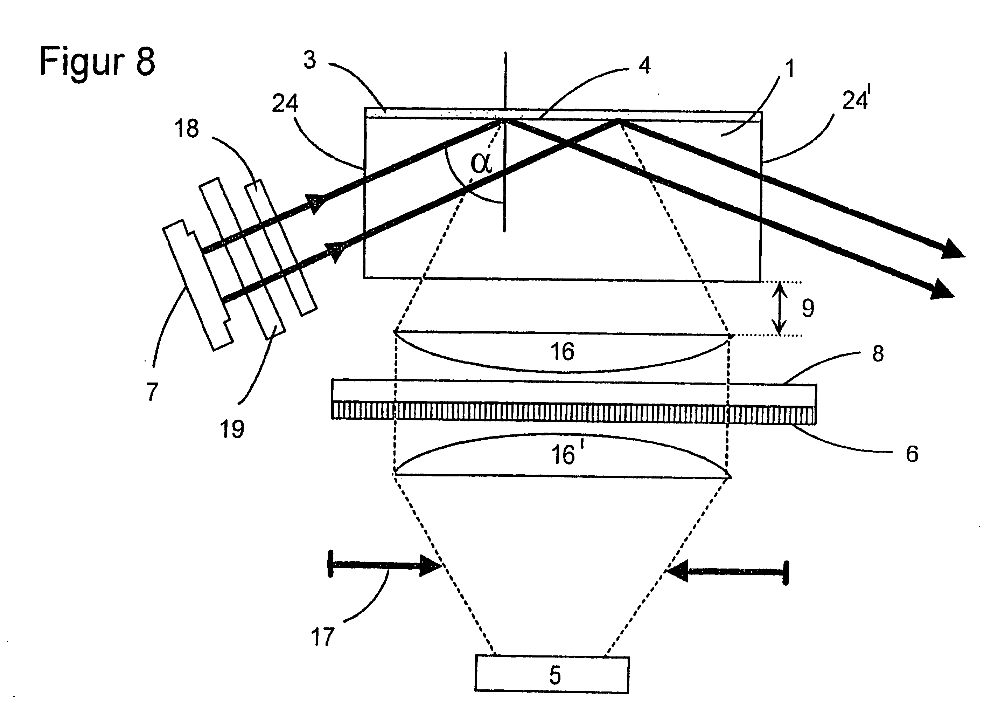

Der Abstand 9 zwischen dem transparenten Körper 25 bzw. der Bodenplatte 1, für den Fall, daß diese entsprechend Figur 8 einteilig ausgebildet ist, und der Linse 16 sollte im Bereich zwischen 0 bis ca. 1000 mm liegen.

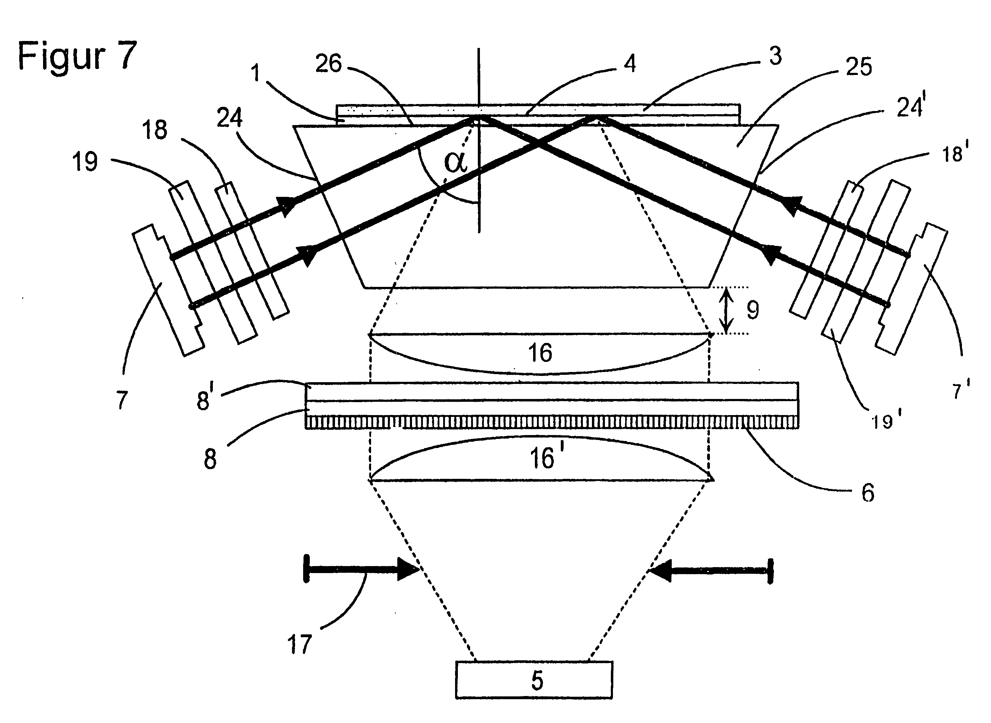

Das in der Figur 7 dargestellte Beispiel einer erfindungsgemäß ausgebildeten Vorrichtung entspricht im wesentlichen dem vorab beschriebenen, in der Figur 6 gezeigten Beispiel. Hierbei ist zusätzlich lediglich eine zweite Lichtquelle 7', ein Filter 19' und ein Polarisator 18' vorhanden. Die Lichtquelle 7' sendet Licht einer Wellenlänge, die sich von der ersten Lichtquelle 7 unterscheidet. Auch bei diesem Beispiel wird vorzugsweise polarisiertes Licht verwendet. Die in der Figur 7 gezeigte Vorrichtung kann vorteilhaft eingesetzt werden, wenn unterschiedliche Markierungsstoffe, die bei verschiedenen Wellenlängen angeregt werden können, verwendet werden. Beispiele hierfür sind die Fluorophore Cy5 und Cy7. Dabei wird zur Anregung des Fluorophor Cy5 eine Laserdiode mit einem Licht einer Wellenlänge zwischen 635 und 655 nm und für die Fluorophore Cy7 eine Laserdiode, die Licht mit einer Wellenlänge zwischen 730 bis 780 nm sendet, verwendet.

Die Messung erfolgt bei dieser Ausführungsform in der Weise, daß die Dioden 7, 7' entweder alternierend geschaltet oder beispielsweise entsprechend synchronisierte Chopper eingesetzt werden, so daß gesichert ist, daß jeweils nur Licht einer Lichtquelle 7 oder 7' zur Anregung auf die Probe gelangen kann und dadurch keine Verfälschungen auftreten.

Da aber hierbei zwei verschiedene Fluoreszenzsignale das gleiche Filter passieren müssen, kann ein breitbandiges Filter 8 nicht mehr verwendet werden. Es sollten daher zwei Filter 8, 8' nacheinander angeordnet werden, die selektiv die Wellenlängen der anregenden Lichtquellen 7,7' sperren. Hierfür können z.B. Notch-Filter verwendet werden. Eine weitere Möglichkeit besteht darin, entsprechend synchronisiert mit den Laserdioden-Chopper oder dem An- und Ausschalten das entsprechende Filter mechanisch in den Strahlengang zwischen den Linsen 16 und 16' zu bringen bzw. aus diesem zu entfernen. Eine weitere Möglichkeit besteht darin, die Lichtquellen 7, 7' kontinuierlich angeschaltet zu lassen und die entsprechenden Filter 8, 8' alternierend mechanisch in den Strahlengang zwischen den Linsen 16, 16' zu bringen oder aus diesen zu entfernen.

Mit dieser Anordnung kann einmal ein Referenzsignal erhalten werden, das eine interne Kalibrierung des Meßsignals ermöglicht. Zur Referenzmessung wird ein Referenzantikörper, der nicht gegen ein Antigen aus der Probe gerichtet ist, verwendet. Der Referenzantikörper ist vorab quantifiziert und mit einem unterschiedlichen Markierungsstoff vom zu bestimmenden analytspezifischen Antikörper Ak unterscheidbar gemacht. Die Menge der an die Oberfläche tatsächlich gebundenen Referenzantikörper kann mit einer zweiten Lichtquelle 7', die Licht einer Fluoreszenz des unterschiedlichen Markierungsstoffes hervorruft, einem zweiten Streulichtfilter 8' und dem Detektor 5 bestimmt werden. Mit dieser Bestimmung können die Verluste der nicht an die Oberfläche gebundenen markierten analytspezifischen Antikörper Ak bzw. Antigene Ag berücksichtigt werden.

Neben der Gewinnung eines Referenzsignales können aber auch zwei unabhängig voneinander laufende Immunotests durchgeführt werden, wobei die Unterscheidung an Hand der unterschiedlichen Fluorophore erfolgt.

Vorteilhaft kann eine lineare oder flächenhafte Anordnung von lichtempfindlichen Detektoren als Detektor 5 verwendet werden. Dadurch können parallel mehrere Analyte detektiert werden, wenn im Aufnahmebereich 2 an unterschiedlichen Orten je nach biochemischen Assay entweder unterschiedliche Antigene beim kompetitiven Assay oder unterschiedliche anatlytspezifische Antikörper beim Sandwichassay immobilisiert werden und entsprechend der Menge der unterschiedlichen Antigene oder Antikörper unterschiedlich markierte analytspezifische Antikörper sich im Probencontainer 10 befinden. Die unterschiedlich markierten analytspezifischen Antikörper werden entsprechend des biochemischen Assay an unterschiedlichen Orten im Aufnahmebereich 2 anbinden und durch die Abbildung des Fluoreszenzlichtes mit Hilfe des Kollimators 16 auf die lineare oder flächenhafte Anordnung von mehreren lichtempfindlichen Detektoren wird das Fluoreszenzlicht ortsaufgelöst detektiert. Dies ermöglicht bei der Verwendung von nur einem Markierungsstoff, wie z.B. Cy5 die unabhängige und parallele Quantifizierung von mehreren Analyten aus einer Probe. Der in den Figuren dargestellte Detektor 5 wird dann durch eine entsprechende Anordnung mehrerer lichtempfindlicher Detektoren gebildet.

Das in der Figur 8 gezeigte Ausführungsbeispiel entspricht im wesentlichen dem bereits bei der Beschreibung der Figur 6 erklärten Beispiel. Hierbei ist jedoch der Aufbau etwas vereinfacht worden, in dem die Bodenplatte 1 die Aufgaben des transparenten Körpers 25 übernimmt und entsprechend größer, dimensioniert ist.

Die bei diesem Beispiel gezeigte Bodenplatte 1 hat einen rechteckigen Querschnitt, mit dem zwar Einkoppelverluste hingenommen werden müssen, sich jedoch der Fertigungsaufwand verringert. Selbstverständlich kann die Bodenplatte 1 aber auch so ausgebildet sein, wie dies beim in der Figur 6 gezeigten transparenten Körper 25 der Fall ist und die Stirnflächen 24 bzw. 24' so geneigt sind, daß das Licht der Laserdiode 7 orthogonal auftreffen kann.

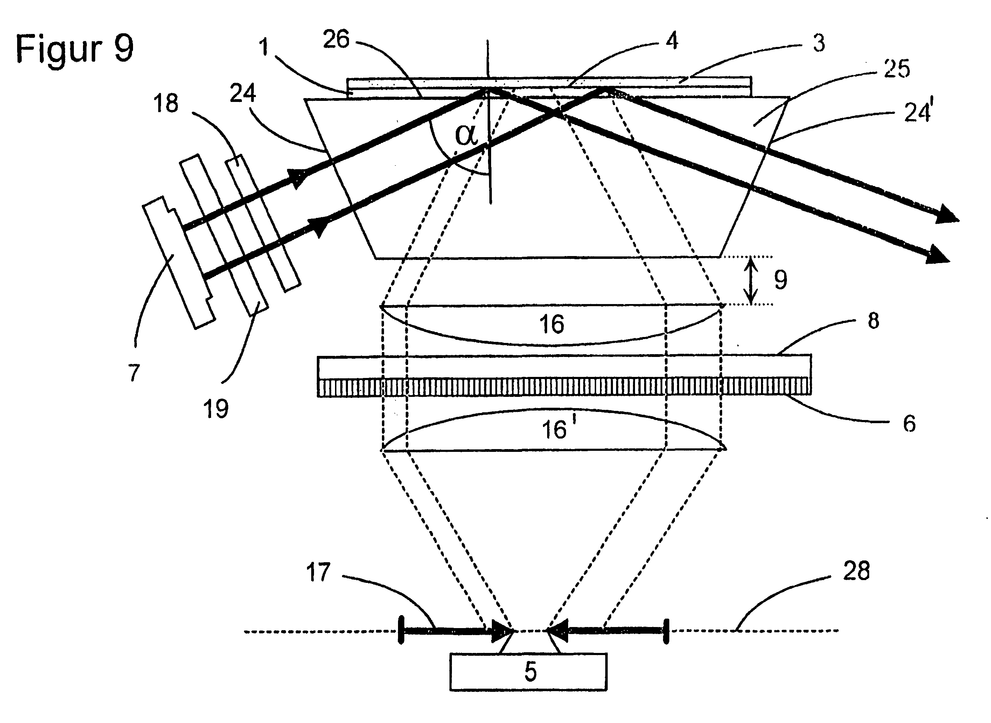

In der Figur 9 ist ein weiteres die Erfindung weiterbildendes Ausführungsbeispiel dargestellt. Obwohl beim größten Teil des dargestellten Aufbaus auf das in der Figur 6 gezeigte Ausführungsbeispiel zurückgegriffen worden ist, können aber auch die anderen Ausführungen entsprechend ergänzt werden.

Bei diesem Beispiel wird eine Blende 17 verwendet, die in bezug auf den Detektor 5 und die Bodenplatte 1 bewegbar ist.

Die Öffnungsabmaße der Blende 17 können je nach Anwendung angepaßt sein, so daß kreisförmige, ovale oder schlitzförmige Aussparungen für die Blende 17 einsetzbar sind. Ein weiteres Auswahlkriterium kann auch die Größe des freien Blendenquerschnitts sein.

Bei konstantem freien Querschnitt der Blende 17, kann es günstig sein, die Blende 17 bzw. den Detektor 5 so zu gestalten, daß der Abstand zueinander verändert werden kann. Eine weitere Möglichkeit besteht darin, einen drehbaren Körper vorzusehen, in dem mehrere verschiedene Blenden 17 vorhanden sind, die sich in Größe und/oder Lage unterscheiden, so daß verschiedene Bereiche des Aufnahmebereiches 2 abgebildet und mit dem Detektor 5 ortsaufgelöst erfaßt werden können.

Da mit Verwendung der Blende 17 nur ein Teil des Fluoreszenzlichtes auf dem Detektor 5 abgebildet wird, ist in der Figur 9 eine verschiebbare Blende 17 dargestellt.

Vorteilhaft ist die Blende 17 in die Bildebene 28 des abbildenden Linsensystems aus den Halblinsen 16 und 16' angeordnet und vor dem Detektor 5 im Strahlengang des Fluoreszenzlichtes angeordnet.

Durch Verschieben der Blende 17 kann die Oberfläche des Aufnahmebereiches 2 sequentiell abgetastet werden.

Bei der Durchführung von biochemischen Assays, bei denen an unterschiedlichen Orten im Aufnahmebereich 2 unterschiedliche oder gleiche chemische oder biochemische Komponenten immobilisiert sind und entsprechend der jeweiligen sich dort befindenden Anzahl der immobilisierten Komponenten komplementäre markierte chemische oder biochemische Substanzen im dargestellten Probencontainer 10 enthalten sind, kann die Anbindung der komplementären und markierten Substanzen ortsaufgelöst erfaßt werden. Dies erfolgt dadurch, daß die Blende 17 parallel und/oder orthogonal zur Bildebene bewegt wird, wobei damit flächendeckend der gesamte Aufnahmebereich 2 abgefahren wird.

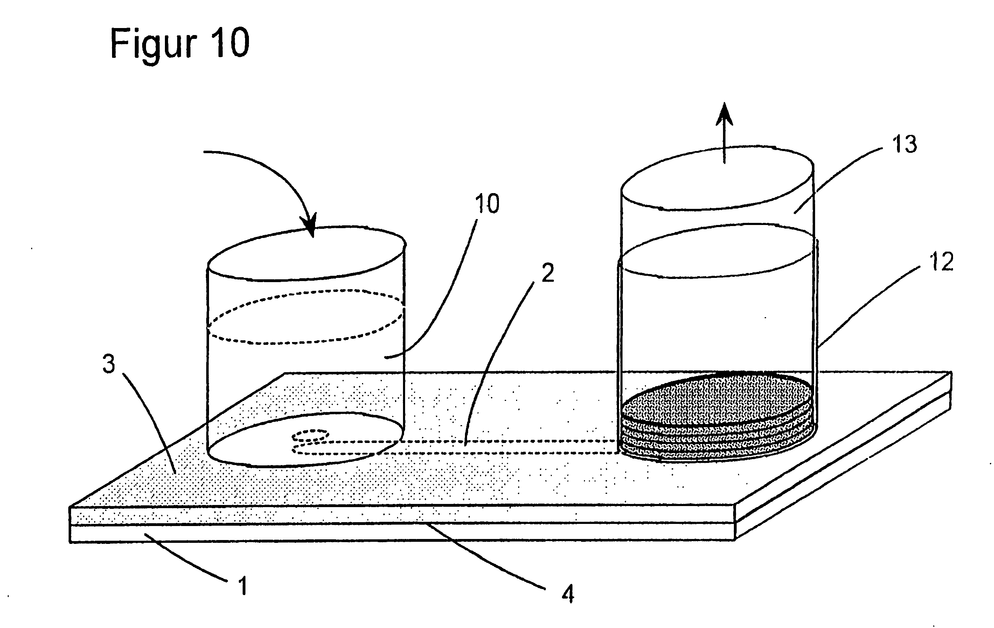

In der Figur 10 ist dargestellt, wie ein Probencontainer 10 zur Öffnung 9 in der Deckplatte 3 angeordnet ist und so eine Verbindung zwischen Probencontainer 10 über Öffnung 9 zum Aufnahmebereich 2 herstellbar ist. Dabei bildet der Probencontainer 10 den Behälter, in dem die bekannte Menge mit dem Markierungsstoff Fluorophor markierter Antikörper Ak in der zu bestimmenden Probe vermischt werden. Der Probencontainer 10 sollte vorteilhaft immer mit der gleichen Menge befüllt werden, um reproduzierbare Ergebnisse erhalten zu können. Günstig sollte er dabei immer maximal befüllt werden. Bei allen durchführbaren Assayformaten kann sich der spezifische Antikörper Ak jeweils auf der Oberfläche des Probencontainers 10 befinden und durch den Kontakt mit der flüssigen Probe löst sich dieser von der Oberfläche und gelangt in die Probe. Eine einfache und bereits bekannte Methode besteht darin, lyophilisierte Antikörper auf die Oberfläche des Probencontainers 10 aufzubringen. Dadurch wird es möglich, den Test relativ lange vor der eigentlichen Durchführung des Immunotests zu lagern. Der Aufnahmebereich 2 definiert die Oberfläche auf der Bodenplatte 1, auf der je nach Assayformat die jeweils entsprechenden chemischen oder biochemischen Substanzen immobilisiert werden.

In der Figur 10 ist ebenfalls ein bevorzugt zylinderförmiger Hohlkörper 12, in dem ein Kolben 13 oder eine andere geeignete Abdeckung (Stöpsel, Kappe, Folie) aufgenommen ist, dargestellt, die beide zusammen als Pumpe dienen. Wird der Kolben 13 aus dem zylinderförmigen Hohlkörper 12 herausbewegt, entsteht ein Unterdruck, der Probenmaterial aus dem Probencontainer 10 durch den Aufnahmebereich 2 in Richtung auf den zylinderförmigen Hohlkörper 12 saugt. Durch Kapillarkräfte im Aufnahmebereich 2 und durch ein Flüssigkeit aufnehmendes Material, auf dem Boden des zylinderförmigen Hohlkörpers 12, wird der Fluß aufrechterhalten, bis das gesamte Probenvolumen durch den Aufnahmebereich 2 gefördert worden ist. Der zylinderförmige Hohlkörper 12 ist aufgesetzt oder hat ein Loch im Boden, so daß eine Verbindung zum Aufnahmebereich 2 vorhanden ist. Dies kann durch die zweite Öffnung 11 als Anschlußmöglichkeit in der Deckplatte 3 realisiert werden. Wird keine Deckplatte 3 verwendet, kann die Anschlußmöglichkeit auch anders ausgebildet sein.

Es kann aber auch an die Öffnung 11 eine externe andere Pumpe angeschlossen werden.

Nach Aufbringen der Probe (mit dem Probencontainer 10) muß eine entsprechende Zeit gewartet werden, so daß die gewünschte Bindung zwischen den Antigenen Ag und den markierten Antikörpern Ak vollständig erfolgen kann. Im Anschluß daran wird die Pumpe 12, 13 aktiviert und es wird gewartet, bis die gesamte Flüssigkeit durch den Aufnahmebereich 2 gepumpt worden ist. Nach Anregung mit der Lichtquelle 7 bzw. den Lichtquellen 7 und 7' kann dann die Antigenkonzentration bestimmt werden, wobei der erfindungsgemäße Aufbau, wie er in den Figuren 6 und 7 dargestellt worden ist, angewendet werden soll.

Der Aufbau, wie er bisher dargestellt und beschrieben worden ist, kann für die verschiedensten biochemische Assay's genutzt werden.

Beim kompetitiven Assay befinden sich im Probencontainer 10 nicht immobilisierte mit einem Fluorophor markierte analytspezifische Antikörper Ak und im Aufnahmebereich 2 auf der Bodenplatte 1, die aus dem hochbrechenden Glas oder einem Polymer oder anderem geeigneten Kunststoff besteht, ist das entsprechende Antigen Ag immobilisiert.

Die Probe wird dann in den Probencontainer 10 gefüllt und der Analyt bindet sich an den markierten Antikörper Ak. Nach der Reaktion wird die Pumpe 12, 13 aktiviert und die noch freien markierten Antikörper binden an das immobilisierte Antigen Ag im Aufnahmebereich 2 auf der Bodenplatte 1. Die entsprechende Menge an markierten analytspezifischen Antikörpern Ak mit der Messung der Fluoreszenzintensität kann quantifiziert werden, wie dies bereits beschrieben worden ist.

Ein weiteres biochemisches Assay, kann wie folgt durchgeführt werden. Auf dem Boden des Probencontainers 10 befindet sich eine Membran (nicht dargestellt), auf der das Antigen Ag immobilisiert ist und an den Wänden des Probencontainers 10 befinden sich lyophilisierte mit einem Fluorophor markierte analytspezifische Antikörper Ak. An der Oberfläche der Bodenplatte 1, die aus einem bereits bezeichneten Material besteht, ist z.B. ein anti-Antikörper oder Protein A, der gegen den analytspezifischen Antikörper Ak gerichtet ist, immobilisiert. Die Probe wird dann in den Probencontainer 10 gefüllt und die Antikörper Ak binden an den Analyten. Nach der Reaktion wird die Pumpe 12, 13 aktiviert und die noch freien Antikörper Ak binden an die Antigene Ag auf der Membran. Die analytgebundenen Antikörper werden auf der Oberfläche im Aufnahmebereich 2 gebunden und die entsprechende Menge kann dann, wie bereits beschrieben, unter Ausnutzung der Evaneszenzfeldanregung quantifiziert werden.

Wichtig ist bei allen biochemischen Assays, daß sich im Probencontainer 10 ein relativ großes Probenvolumen befindet, das vollständig an der kleinen, durch den Aufnahmebereich 2 gebildeten Meßoberfläche vorbei gepumpt wird. Da die Höhe des Aufnahmebereiches 2 verhältnismäßig klein ist, kann davon ausgegangen werden, daß die entsprechenden Antikörper Ak (freie beim kompetitiven Assay und gebundene beim Sandwichassay) mit Sicherheit durch die Prozesse Konvektion und Diffusion an die Oberfläche gelangen und dies über einen großen Bereich an Flußgeschwindigkeiten der Fall ist. Dadurch kann einmal eine Anreicherung der Antikörper Ak an der Oberfläche und zum anderen eine stabile Betriebssicherheit erreicht werden, so daß der Prozeß nahezu unabhängig von der Geschwindigkeit der Durchströmung ist.

In der Figur 11 ist ein weiteres mögliches Ausführungsbeispiel der Vorrichtung wiedergegeben, mit dem biochemische Assay's durchgeführt werden können, wie das bereits bei der Beschreibung der Figur 4 prinzipiell beschrieben worden ist.

Dabei wird zusätzlich eine in den Probencontainer 10 einpreßbare Hülse 15 verwendet. Die Hülse 15 ist nach unten mit einer Membran 23 abgeschlossen. Auf der Oberfläche der Innenseite der Hülse 15 befindet sich der lyophilisierte analytspezifische Antikörper und ggf. der Referenz-Antikörper und auf der Membranoberfläche ist das entsprechende Antigen immobilisiert. In diesem Zustand kann das Ganze über eine längere Zeit gelagert werden. Für die Durchführung dieses Tests nach den Figuren 4 und 9, wird bevorzugt die in der Figur 7 dargestellte Vorrichtung eingesetzt, wobei die unterschiedlichen Antikörper Ak bzw. der Referenz-Antikörper mit unterschiedlichen Fluorophoren markiert werden. Die Quantifizierung erfolgt dann in der bereits beschriebenen Form.

Auch bei dem in Figur 11 gezeigten Beispiel kann wieder ein zylinderförmiger Hohlkörper 12 mit eingesetztem Kolben 13 verwendet werden, wie dies bereits bei dem in Figur 8 gezeigten Beispiel beschrieben worden ist.

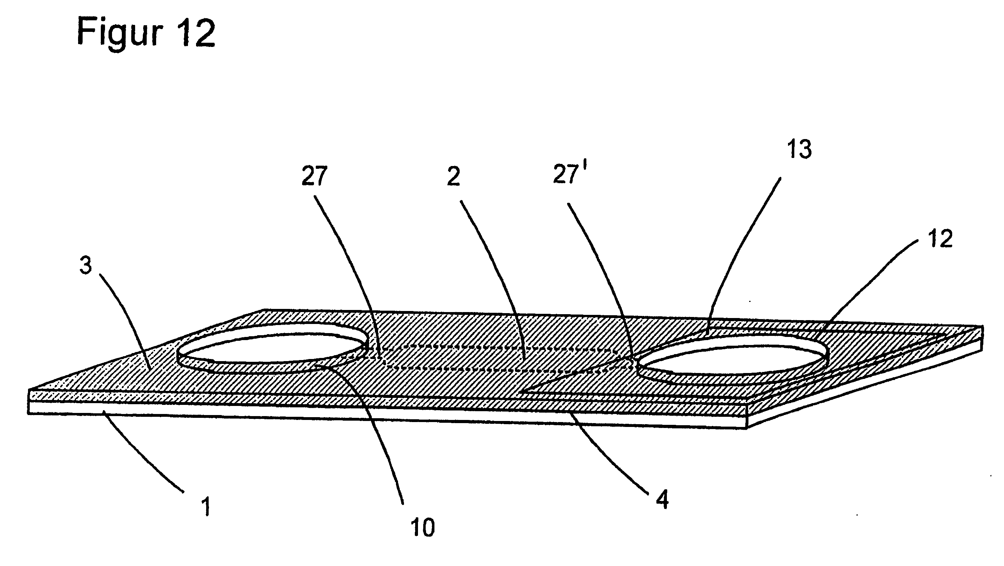

Die Figur 12 zeigt eine weitere Ausbildung der erfindungsgemäßen Vorrichtung, bei dem beidseitig am Aufnahmebereich 2 Verbindungen 27 und 27' ausgebildet sind, durch die die zu bestimmenden chemischen oder biochemischen Substanzen in den Aufnahmebereich 2 eingeführt und wieder abgeführt werden können, wie dies bereits bei anderen Beispielen beschrieben worden ist. Die in der Figur 12 gezeigte Ausbildung des Abstandshalters 4 kann wieder auf einfache Art und Weise durch Ausstanzen erfolgen, wobei alles in einem Stanzvorgang erfolgen kann. Zusätzlich ist die Öffnung 12 des zylindrischen Hohlkörpers 12, der als Ganzes in die Deckplatte 3 eingearbeitet ist und auf diesem Boden sich wieder ein Flüssigkeit aufsaugendes Material befindet oder die Öffnung 11 in der sich ein Flüssigkeit aufnehmendes Material befindet, mit einer Folienabdekkung 13 versehen, die im Ausgangszustand die Öffnung 11 bzw. die Öffnung des zylindrischen Hohlkörpers 12 luftdicht verschließt und durch einfaches Durchstoßen der Abdeckfolie 13 der Fluß der Probe durch den Aufnahmebereich 2 ausgelöst werden kann.

## Patentansprüche

1. Vorrichtung zur Durchführung von quantitativen Fluoreszenz markierten Affinitätstests mittels Evanescentfeldanregung, mit mindestens einer nahezu monochromatisches Licht aussendenden Lichtquelle (7, 7'), die Lichtstrahlen mit einer Fluoreszenz eines an einem chemischen oder biochemischen Partner eines allgemeinen Rezeptor-Ligand Systems gebundenen Markierungsstoffes hervorrufenden Wellenlänge, in einem durch eine vorgebbare Eindringtiefe d für das evanescente Feld bestimmenden Winkel $\alpha$ auf eine Grenzfläche (20) einer optisch transparenten Bodenplatte (1) aus Material dessen Brechungsindex $n_1$ größer ist als der Brechungsindex $n_2$ des Materials oberhalb der Grenzfläche (20) richtet,
**dadurch gekennzeichnet, daß** das Licht durch die Bodenplatte (1) auf die Grenzfläche (20) eines küvettenförmigen Aufnahmebereiches (2), der eine Dicke zwischen 0,001 und 0,5 mm aufweist, gerichtet ist, ein Probencontainer (10) zur Aufnahme der Probe so angeordnet ist, daß eine Verbindung über eine erste Anschlußmöglichkeit (9), die zumindest teilweise eine Öffnung ist, zwischen küvettenförmigem Aufnahmebereich (2) und Probencontainer (10) gebildet ist und eine zweite Anschlußmöglichkeit (11) in Verbindung mit dem küvettenförmigen Aufnahmebereich (2) steht, wobei der Aufnahmebereich (2) auf der entgegengesetzt zur Bodenplatte (1) angeordneten Seite mit einer Deckplatte (3) abgedeckt ist, und
ein Detektor (5) zur Erfassung des Fluoreszenzlichtes gleichseitig zur Bodenplatte (1), wie die Lichtquelle (7, 7') angeordnet ist.

2. Vorrichtung nach Anspruch 1,
**dadurch gekennzeichnet, daß** die Bodenplatte (1) und Deckplatte (3) mit einem Abstandshalter (4), in dem der küvettenförmige Aufnahmebereich (2) ausgebildet ist, verbunden sind.

3. Vorrichtung nach Anspruch 2, **dadurch gekennzeichnet, daß** der Abstandshalter (4) ein Klebefilm oder eine Folie ist.

4. Vorrichtung nach einem der Ansprüche 1 bis 3, **dadurch gekennzeichnet, daß** das Licht der Lichtquelle(n) (7, 7') über mindestens eine Stirnfläche (24, 24') der Bodenplatte (1) oder eines mit der Bodenplatte (1) verbundenen optisch transparenten Körpers (25), aus einem Material mit höherem Brechungsindex als das Material oberhalb der Grenzfläche (20), in die Bodenplatte (1) einkoppelbar ist.

5. Vorrichtung nach Anspruch 4,
**dadurch gekennzeichnet, daß** zwischen Bodenplatte 1 und transparentem Körper (25) eine optisch transparenten Schicht (26) ausgebildet ist, deren Brechungsindex mit dem Brechungsindex der Bodenplatte (1) oder des transparenten Körpers (25) übereinstimmt oder der Brechungsindex zwischen dem der Bodenplatte (1) und des transparenten Körpers (25) liegt.

6. Vorrichtung nach einem der Ansprüche 1 bis 5, **dadurch gekennzeichnet, daß** die Lichtquelle(n) (7, 7') eine oder mehrere Laserdiode(n) ist/sind.

7. Vorrichtung nach einem der Ansprüche 1 bis 6, **dadurch gekennzeichnet, daß** die Lichtquelle(n) (7, 7') polarisiertes Licht aussendet/aussenden oder hinter den Lichtquelle(n) (7, 7') ein Polarisator (18, 18') und vor dem Detektor (5) ein Polarisator (6) angeordnet ist.

8. Vorrichtung nach einem der Ansprüche 1 bis 7, **dadurch gekennzeichnet, daß** im Strahlengang direkt hinter der/den Lichtquelle(n) (7, 7') jeweils ein optisches Filter (19, 19') angeordnet ist.

9. Vorrichtung nach einem der Ansprüche 1 bis 8, **dadurch gekennzeichnet, daß** vor dem Detektor (5) mindestens ein optisches Filter (8, 8') angeordnet ist.

10. Vorrichtung nach einem der Ansprüche 1 bis 9, **dadurch gekennzeichnet, daß** die optischen Filter (8, 8') alternierend in den Strahlengang zwischen der Bodenplatte (1) und dem Detektor (5) einführbar und entfernbar sind.

**11.** Vorrichtung nach einem der Ansprüch 1 bis 10, **dadurch gekennzeichnet, daß** in den Strahlengängen Chopper angeordnet ist/sind.

**12.** Vorrichtung nach einem der Ansprüche 1 bis 11, **dadurch gekennzeichnet, daß** der Detektor (5) eine lineare oder flächige Anordnung mehrerer lichtempfindlicher Detektoren ist.

**13.** Vorrichtung nach einem der Ansprüche 1 bis 12, **dadurch gekennzeichnet, daß** im Strahlengang vor dem Detektor (5) mindestens eine Linse (16, 16') angeordnet ist/sind.

**14.** Vorrichtung nach einem der Ansprüche 1 bis 13, **dadurch gekennzeichnet, daß** im Strahlengang zwischen Bodenplatte (1) und dem Detektor (5) eine Blende (17) angeordnet ist.

**15.** Vorrichtung nach Anspruch 14, **dadurch gekennzeichnet, daß** die Blende (17) bewegbar ist.

**16.** Vorrichtung nach Anspruch 1, **dadurch gekennzeichnet, daß** eine zweite Lichtquelle (7') Licht einer Fluoreszenz eines zweiten Markierungsstoffes hervorrufenden Wellenlänge sendend vorhanden ist, und
das Licht beider Lichtquellen (7, 7') alternierend auf den Aufnahmebereich (2) richtbar ist.

**17.** Vorrichtung nach einem der Ansprüche 1 bis 16, **dadurch gekennzeichnet, daß** der/die Markierungsstoff(e) Fluorophore ist/sind.

**18.** Vorrichtung nach einem der Ansprüche 1 bis 17, **dadurch gekennzeichnet, daß** die Bodenplatte (1) aus optisch transparentem Material, wie Glas oder einem Kunststoff besteht.

**19.** Vorrichtung nach einem der Ansprüche 1 bis 18, **dadurch gekennzeichnet, daß** an die zweite Anschlußmöglichkeit (11) eine Pumpe anschließbar oder in diese einsetzbar ist.

**20.** Vorrichtung nach Anspruch 18, **dadurch gekennzeichnet, daß** ein als Pumpe wirkender Hohlkörper (12) mit einer Abdeckung (13) verwendbar ist.

**21.** Vorrichtung nach Anspruch 20, **dadurch gekennzeichnet, daß** im Boden des Hohlkörpers (12) ein Flüssigkeit aufsaugendes Material (14) angeordnet ist.

**22.** Vorrichtung nach einem der Ansprüche 1 bis 21, **dadurch gekennzeichnet, daß** in den Probencontainer (10) ein Hohlkörper (15), an dessen Innenwandung sich Antikörper befinden, einsetzbar ist.

**23.** Vorrichtung nach Anspruch 22, **dadurch gekennzeichnet, daß** der Hohlkörper (15) nach unten mit einer Membran (23), auf der Antigene immobilisiert sind, abgeschlossen ist.

**24.** Verfahren zur Durchführung von Fluoreszenzimmunotests mittels Evanescentfeldanregung, **dadurch gekennzeichnet, daß** ein Probenvolumen aus einem Probencontainer (10) mittels Saug-, Druckwirkung oder Kappilarkräften durch einen küvettenförmigen Aufnahmebereich (2) hindurch geführt wird und die je nach biochemischen Assay zu bestimmenden markierten chemischen oder biochemischen Komponenten an den entsprechenden komplementären chemischen oder biochemischen Komponenten, die an der Oberfläche im Aufnahmebereich (2) immobilisiert sind gebunden werden und durch Evanscentfeldanregung mittels der Lichtquelle(n) (7, 7') mit einem Detektor (5) das Fluoreszenzlicht gemessen wird.

**25.** Verfahren nach Anspruch 24, **dadurch gekennzeichnet, daß** eine Referenzmessung mit einer chemischen oder biochemischen Referenzkomponente, die nicht einen Analyt aus der Probe bestimmt und eine unterschiedliche Markierung gegenüber der chemischen oder biochemischen Komponente zum Nachweis des Analyten hat, die Menge der chemischen oder biochemischen Referenzkomponente durch die zweite Lichtquelle (7') und einem zweiten optischen Filter (8') mit dem Detektor (5) quantifizierbar ist, durchgeführt wird.

**26.** Verfahren nach Anspruch 24, **dadurch gekennzeichnet, daß** zwei verschiedene an unterschiedliche Markierungsstoffe gebundene markierte chemische oder biochemische Komponenten mit zwei Lichtquellen (7, 7'), die Licht mit Wellenlängen, die Fluoreszenz des jeweiligen Markierungsstoffes hervorrufen, so daß zwei unterschiedliche Analyte aus einer Probe mit der Detektion der jeweiligen Fluoreszenzintensität quantifiziert werden.

**27.** Verfahren nach einem der Ansprüche 24 bis 26, **dadurch gekennzeichnet, daß** zur Bestimmung der Fluoreszenzintensitäten unterschiedlicher Markierungen, die Strahlen der Lichtquellen (7, 7') alternierend auf den küvettenförmigen Aufnahmebereich (2) gerichtet werden oder die Lichtstrahlen der Lichtquellen (7, 7') kontinuierlich auf den küvettenförmigen Aufnahmebereich (2) gerichtet sind und die Filter (8, 8') alternierend in den Strahlengang zwischen Bodenplatte (1) und Detektor (5) eingeführt und entfernt werden

**28.** Verfahren nach Anspruch 24, **dadurch gekennzeichnet, daß** auf der Oberfläche im küvettenförmigen Aufnahmebereich (2) entsprechend dem gewählten biochemischen Assay, an unterschiedlichen Orten unterschiedliche oder gleiche chemische oder biochemische Komponenten immobilisiert und entsprechend der Anzahl der immobilisierten komponenten komplementäre chemische oder biochemische Komponenten im Probencontainer (10) enthalten sind, wobei durch Detektion der unterschiedlichen Fluoreszenzintensitäten der unterschiedlichen Orte mit einem Detektor (5) aus linear oder flächenhaft angeordneten lichtempfindlichen Detektoren die Menge der unterschiedlichen Analyte aus der Probe quantifiziert wird.

**29.** Verfahren nach Anspruch 24, **dadurch gekennzeichnet, daß** auf der Oberfläche im küvettenförmigen Aufnahmebereich (2) entsprechend dem gewählten biochemischen Assay, an unterschiedlichen Orten unterschiedliche oder gleiche chemische oder biochemische Komponenten immobilisiert und entsprechend der Anzahl der immobilisierten Komponenten komplementäre chemische oder biochemische Komponenten im Probencontainer (10) enthalten sind, und die Fluoreszenzintensitäten der unterschiedlichen Orte durch Bewegung der Blende (17), deren Öffnungsabmaße an die gewünschte geometrische Auflösung angepaßt sind, ortsaufgelöst über die gesamte Fläche des küvettenförmigen Aufnähmebereiches (2) mit dem Detektor (5) erfaßt und damit die Menge der unterschiedlichen Analyte aus der Probe quantifiziert wird.

**Claims**

**1.** Device for carrying out quantitative fluorescence-marked affinity tests by means of evanescent field excitation, having at least one light source (7, 7') which emits almost monochromatic light and directs light beams, having a wavelength which causes fluorescence of a marking substance bound to a chemical or biochemical partner of a general receptor/ligand system, at an angle $\alpha$ to be defined by a predeterminable penetration depth d for the evanescent field onto an interface (20) of an optically transparent baseplate (1) made of a material whose refractive index $n_1$ is greater than the refractive index $n_2$ of the material above the interface (20), **characterized in that** the light is directed through the baseplate (1) onto the interface (20) of a reception region (2) which is in the form of a cuvette and has a thickness of between 0.001 and 0.5 mm, a sample container (10) for receiving the sample is arranged in such a way that a link is formed via a first possible connection (9), which is at least partially an opening between the reception region (2) in the form of a cuvette and the sample container (10) and a second possible connection (11) is linked to the reception region (2) in the form of a cuvette, the reception region (2) being covered on the opposite side from the baseplate (1) with a cover plate (3), and a detector (5) for picking up the fluorescent light being arranged on the same side of the baseplate (1) as the light source (7, 7').

**2.** Device according to claim 1, **characterized in that** the baseplate (1) and the cover plate (3) are linked with a spacer (4) in which the reception region (2) in the form of a cuvette is made.

**3.** Device according to claim 2, **characterized in that** the spacer (4) is an adhesive film or a sheet.

**4.** Device according to one of claims 1 to 3, **characterized in that** the light from the light source(s) (7, 7') can be coupled into the baseplate (1) via at least one end face (24, 24') of the baseplate (1) or an optically transparent body (25) which is linked to the baseplate (1) and is made of a material having a higher refractive index than the material above the interface (20).

**5.** Device according to claim 4, **characterized in that** an optically transparent layer (26), whose refractive index corresponds to the refractive index of the baseplate (1) or of the transparent body (25) of the refractive index lies between that of the baseplate (1) and that of the transparent body (25), is formed between the baseplate (1) and the transparent body (25).

**6.** Device according to one of claims 1 to 5, **characterized in that** the light cource(s) (7, 7') is/are one or more laser diode(s).

**7.** Device according to one of claims 1 to 6, **characterized in that** the light source(s) (7, 7') emits/emit polarized light, or a polarizer (18, 18') is arranged behind the light source(s) (7, 7') and a polarizer (6) is arranged in front of the detector (5).

**8.** Device according to one of claims 1 to 7, **characterized in that** an optical filter (19, 19') is arranged in the optical path directly behind the one or each light source (7, 7').

**9.** Device according to one of claims 1 to 8, **characterized in that** at least one optical filter (8, 8') is arranged in front of the detector (5).

**10.** Device according to one of claims 1 to 9, **characterized in that** the optical filters (8, 8') can alternately be moved into and out of the optical path between the baseplate (1) and the decector (5).

**11.** Device according to one of claims 1 to 10, **characterized in that** chopper(s) is/are arranged in the optical paths.

**12.** Device according to one of claims 1 to 11, **characterized in that** the detector (5) is a one-dimensional or two-dimensional arrangement of a plurality of photosensitive detectors.

**13.** Device according to one of claims 1 to 12, **characterized in that** at least one lens (16, 16') is/are arranged in the optical path in front of the detector (5).

**14.** Device according to one of claims 1 to 13, **characterized in that** a diaphragm (17) is arranged in the optical path between the baseplate (1) and the detector (5).

**15.** Device according to claim 14, **characterized in that** the diaphragm (17) can be moved.

**16.** Device according to claim 1, **characterized in that** a second light source (7') delivering light having a wavelength which can cause fluorescence of a second marking substance is present, and the light from the two light sources (7, 7') can alternately be directed onto the reception region (2).

**17.** Device according to one of claims 1 to 16, **characterized in that** the marking substance(s) is/are fluorophores.

**18.** Device according to one of claims 1 to 17, **characterized in that** the baseplate (1) is made of an optically transparent material, such as glass or a plastic.

**19.** Device according to one of claims 1 to 18, **characterized in that** a pump can be connected to or fitted into the second possible connection (11).

**20.** Device according to claim 18, **characterized in that** use can be made of a hollow body (12) acting as a pump with a cover (13).

**21.** Device according to claim 20, **characterized in that** a material (14) which absorbs liquid is arranged in the bottom of the hollow body (12).

**22.** Device according to one of claims 1 to 21, **characterized in that** a hollow body (15), on whose inner wall there are antibodies, can be fitted into the sample container (10).

**23.** Device according to claim 22, **characterized in that** the hollow body (15) is closed off at the bottom with a membrane (23) on which antigens are fixed.

**24.** Method for carrying out fluorescence immuno tests by means of evanescent field excitation, **characterized in that** a sample volume is taken from a sample container (10) through a reception region (2) in the form of a cuvette by suction, pressure or capillary forces and the marked chemical or biochemical components which are to be determined according to the biochemical assay are bound to the corresponding complementary chemical or biochemical components which are fixed on the surface in the reception region (2), and the fluorescent light is measured with a detector (5) by evanescent field excitation by means of the light source(s) (7, 7').

**25.** Method according to claim 24, **characterized in that** a reference measurement is carried out with a chemical or biochemical reference component which does not determine an analyte from the sample and which has different marking than the chemical or biochemical component for registering the analyte,
the amount of chemical or biochemical reference component can be quantified by the second light source (7') and a second optical filter (8') with a detector (5).

**26.** Method a according to claim 24, **characterized in that** two different marked chemical or biochemical components, bound to different marking substances,
with two light sources (7, 7'),
which light with wavelengths that cause fluorescence of the respective marking substance, so that two different analytes from a sample are quantified with the detection of the respective fluorescent intensity.

**27.** Method according to one of claims 24 to 26, **characterized in that**, in order to determine the fluorescent intensities of different markings, the beams from the light sources (7, 7') are alternately directed onto the reception region (2) in the form of a cuvette, or the light beams from the light sources (7, 7') are continuously directed onto the reception region (2) in the form of a cuvette and the filters (8, 8') are alternately moved into and out of the optical path between the baseplate (1) and the detector (5).

**28.** Method according to claim 24, **characterized in that** different or the same chemical or biochemical components are fixed on the surface in the reception region (2) in the form of a cuvette according to the chosen biochemical assay, at different locations, and complementary chemical or biochemical components are contained in the sample container (10) in accordance with the number of fixed components, the amount of different analytes from the sample being quantified by detecting the different fluorescent intensities of the different locations with a detector (5) made up of one-dimensionally or two-dimensionally arranged photosensitive detectors.

29. Method according to claim 24, **characterized in that** different or the same chemical or biochemical components are fixed on the surface in the reception region (2) in the form of a cuvette according to the chosen biochemical assay, at different locations, and complementary chemical or biochemical components are contained in the sample container (10) in accordance with the number of fixed components, and the fluorescent intensities of the different locations are picked up with spatial resolution over the entire area of the reception region (2) in the form of a cuvette with the detector (5), by moving the diaphragm (17) whose aperture dimensions are matched to the desired geometrical resolution, and the amount of different analytes from the sample are thereby quantified.

**Revendications**

1. Dispositif pour mettre en oeuvre des tests quantitatifs d'affinité à fluorescence au moyen d'une excitation de champ évanescent, comprenant au moins une source de lumière (7, 7') émettant de la lumière presque monochromatique, qui dirige des rayons de lumière avec une longueur d'onde provoquant une fluorescence d'un matériau de marquage lié à un partenaire chimique ou biochimique d'un système ligand-récepteur général avec un angle α déterminant par une profondeur de pénétration d spécifiable pour le champ évanescent sur une surface interfaciale (20) d'une plaque de fond (1) optiquement transparente composée de matériau dont l'indice de réfraction $n_1$ est supérieur à l'indice de réfraction $n_2$ du matériau au-dessus de la surface interfaciale (20),
    **caractérisé en ce**
    **que** la lumière est dirigée par la plaque de fond (1) sur la surface interfaciale (20) d'une zone de réception en forme de cuve (2) qui présente une épaisseur comprise entre 0,001 et 0,5 mm, un conteneur d'échantillon (10) est disposé en vue de la réception de l'échantillon de manière à ce qu'un composé soit formé par l'intermédiaire d'une première possibilité de raccordement (9), qui est au moins en partie une ouverture, entre la zone de réception en forme de cuve (2) et le conteneur d'échantillon (10) et une seconde possibilité de raccordement (11) est en contact avec la zone de réception en forme de cuve (2), la zone de réception (2) étant recouverte d'une plaque de recouvrement (3) sur la face opposée à la plaque de fond (1), et

un détecteur (5) destiné à l'acquisition de la lumière fluorescente est disposé équilatéralement par rapport à la plaque de fond (1), comme la source de lumière (7, 7').

2. Dispositif selon la revendication 1, **caractérisé en ce que** la plaque de fond (1) et la plaque de recouvrement (3) sont reliées avec un séparateur (4) dans lequel la zone de réception en forme de cuve (2) est formée.

3. Dispositif selon la revendication 2, **caractérisé en ce que** le séparateur (4) est un film adhésif ou une feuille.

4. Dispositif selon l'une quelconque des revendications 1 à 3, **caractérisé en ce que** la lumière de la/des source(s) de lumière (7, 7') est injectable dans la plaque de fond (1) par l'intermédiaire d'au moins une face terminale (24, 24') de la plaque de fond (1) ou d'un corps (25) transparent optiquement relié à la plaque de fond (1) composé d'un matériau avec un indice de réfraction plus élevé que le matériau au-dessus de la surface interfaciale (20).

5. Dispositif selon la revendication 4, **caractérisé en ce qu'**une couche optiquement transparente (26) est réalisée entre la plaque de fond (1) et le corps transparent (25), l'indice de réfraction de ladite couche correspondant à l'indice de réfraction de la plaque de fond (1) ou du corps transparent (25), ou l'indice de réfraction étant compris entre celui de la plaque de fond (1) et celui du corps transparent (25).

6. Dispositif selon l'une quelconque des revendications 1 à 5, **caractérisé en ce que** la/les source(s) de lumière (7, 7') est/sont une ou plusieurs diode(s) laser.

7. Dispositif selon l'une quelconque des revendications 1 à 6, **caractérisé en ce que** la/les source(s) de lumière (7, 7') émet/émettent de la lumière polarisée, ou un polarisateur (18, 18') est disposé après la/les source(s) de lumière (7, 7') et un polarisateur (6) est disposé avant le détecteur (5).

8. Dispositif selon l'une quelconque des revendications 1 à 7, **caractérisé en ce qu'**un filtre optique (19, 19') est disposé à chaque fois dans le trajet des rayons directement après la/les source(s) de lumière (7, 7').

9. Dispositif selon l'une quelconque des revendications 1 à 8, **caractérisé en ce qu'**au moins un filtre optique (8, 8') est disposé avant le détecteur (5).

10. Dispositif selon l'une quelconque des revendications 1 à 9, **caractérisé en ce que** les filtres optiques (8, 8') sont alternativement insérables dans le trajet des rayons entre la plaque de fond (1) et le détecteur (5) et retirables de celui-ci.

11. Dispositif selon l'une quelconque des revendications 1 à 10, **caractérisé en ce qu'**un/des chopper (s) est/sont disposés dans les trajets des rayons.

12. Dispositif selon l'une quelconque des revendications 1 à 11, **caractérisé en ce que** le détecteur (5) est un aménagement linéaire ou plan de plusieurs détecteurs photosensibles.

13. Dispositif selon l'une quelconque des revendications 1 à 12, **caractérisé en ce qu'**au moins une lentille (16, 16') est/sont disposée(s) dans le trajet des rayons avant le détecteur (5).

14. Dispositif selon l'une quelconque des revendications 1 à 13, **caractérisé en ce qu'**un diaphragme (17) est disposé dans le trajet des rayons entre la plaque de fond (1) et le détecteur (5).

15. Dispositif selon la revendication 14, **caractérisé en ce que** le diaphragme (17) est mobile.

16. Dispositif selon la revendication 1, **caractérisé en ce qu'**une seconde source de lumière (7') émettant de la lumière d'une longueur d'onde provoquant une fluorescence d'un second matériau de marquage est disponible, et la lumière des deux sources de lumière (7, 7') est dirigeable alternativement sur la zone de réception (2).

17. Dispositif selon l'une quelconque des revendications 1 à 16, **caractérisé en ce que** le/les matériau (x) de marquage est/sont des fluorophores.

18. Dispositif selon l'une quelconque des revendications 1 à 17, **caractérisé en ce que** la plaque de fond (1) se compose de matériau optiquement transparent, tel que du verre ou un plastique.

19. Dispositif selon l'une quelconque des revendications 1 à 18, **caractérisé en ce qu'**une pompe est raccordable à la seconde possibilité de raccordement (11) ou insérable dans cette dernière.

20. Dispositif selon la revendication 18, **caractérisé en ce qu'**un corps creux (12) agissant en tant que pompe avec un couvercle (13) est utilisable.

21. Dispositif selon la revendication 20, **caractérisé en ce qu'**un matériau (14) absorbant du liquide est disposé dans le fond du corps creux (12).

22. Dispositif selon l'une quelconque des revendications 1 à 21, **caractérisé en ce qu'**un corps creux (15), sur la paroi intérieure duquel se trouvent des anticorps, est insérable dans le conteneur d'échantillon (10).

23. Dispositif selon la revendication 22, **caractérisé en ce que** le corps creux (15) est terminé vers le bas par une membrane (23) sur laquelle sont immobilisés des antigènes.

24. Procédé pour mettre en oeuvre des tests immunologiques fluorescents au moyen d'une excitation de champ évanescent, **caractérisé en ce qu'**un volume d'échantillon provenant d'un conteneur d'échantillon (10) est passé à travers une zone de réception en forme de cuve (2) au moyen d'un effet d'aspiration, d'un effet de pression ou de forces capillaires et les composants chimiques ou biochimiques marqués à déterminer selon l'essai biochimique sont liés aux composants chimiques ou biochimiques complémentaires correspondants qui sont immobilisés sur la surface dans la zone de réception (2) et la lumière fluorescente est mesurée avec un détecteur (5) par excitation du champ évanescent au moyen de la/les source(s) de lumière (7, 7').

25. Procédé selon la revendication 24, **caractérisé en ce qu'**une mesure de référence est réalisée avec un composant chimique ou biochimique de référence, qui ne détermine pas un analyte à partir de l'échantillon et dispose d'un marquage différent vis-à-vis du composant chimique ou biochimique comme justification de l'analyte, la quantité du composant chimique ou biochimique de référence étant quantifiable avec le détecteur (5) par la seconde source de lumière (7') et un second filtre optique (8').

26. Procédé selon la revendication 24, **caractérisé en ce que** deux composants chimiques ou biochimiques marqués différents liés à différents matériaux de marquage avec deux sources de lumière (7, 7'), la lumière avec des longueurs d'onde qui provoquent une fluorescence du matériau de marquage respectif de manière que deux analytes différents provenant d'un échantillon soient quantifiés avec la détection de l'intensité de fluorescence respective.

27. Procédé selon l'une quelconque des revendications 24 à 26, **caractérisé en ce qu'**en vue de la détermination des intensités de fluorescence des différents marquages, les rayons des sources de lumière (7, 7') sont dirigés alternativement sur la zone de réception en forme de cuve (2), ou les rayons de lumière des sources de lumière (7, 7') sont dirigés en continu sur la zone de réception en forme de cuve (2) et les filtres (8, 8') sont alternativement insérés dans le trajet des rayons entre la plaque de fond (1) et le détecteur (5) et retirés de celui-ci.

**28.** Procédé selon la revendication 24, **caractérisé en ce que** des composants chimiques ou biochimiques différents ou identiques sont immobilisés à différents emplacements sur la surface dans la zone de réception en forme de cuve (2) en fonction de l'essai biochimique choisi et en fonction de la quantité de composants immobilisés, des composants chimiques ou biochimiques complémentaires sont contenus dans le conteneur d'échantillon (10), la quantité des différents analytes provenant de l'échantillon étant quantifiée avec un détecteur (5) issu de détecteurs photosensibles disposés linéairement ou planairement par détection des différentes intensités de fluorescence des différents emplacements.

**29.** Procédé selon la revendication 24, **caractérisé en ce que** des composants chimiques ou biochimiques différents ou identiques sont immobilisés sur la surface dans la zone de réception en forme de cuve (2) en fonction de l'essai biochimique choisi à différents emplacements et en fonction de la quantité des composants immobilisés, des composants chimiques ou biochimiques complémentaires sont contenus dans le conteneur d'échantillon (10), et les intensités de fluorescence des différents emplacements acquises avec résolution latérale par mouvement du diaphragme (17) dont les écarts d'ouverture sont adaptés à la résolution géométrique souhaitée, sur toute la surface de la zone de réception en forme de cuve (2) avec le détecteur (5) et, ainsi, la quantité des différents analytes provenant de l'échantillon est quantifiée.

# Figur 1

Figur 2

# Figur 3

# Figur 4

Ak

Ag

22

O

P

Figur 5

# Figur 6

EP 0 910 792 B1

Figur 7

EP 0 910 792 B1

Figur 8

24

Figur 9

Figur 10

EP 0 910 792 B1

Figur 11

EP 0 910 792 B1

Figur 12

EP 0 910 792 B1